# EUROPÄISCHE PATENTSCHRIFT

(11) **EP 2 222 259 B1**
(45) Veröffentlichungstag und Bekanntmachung des Hinweises auf die Patenterteilung: **29.06.2011**
(21) Anmeldenummer: 08858655.7
(22) Anmeldetag: 05.12.2008
(51) Int. Cl.: A61F 9/00, C08F 220/06, C08F 220/18, G02B 1/04

(54) **OPHTHALMOLOGISCHE ZUSAMMENSETZUNG UND DEREN VERWENDUNG**
OPHTHALMOLOGIC COMPOSITION AND USE THEREOF
COMPOSITION OPHTALMOLOGIQUE ET SON UTILISATION

(30) Priorität: 11.12.2007 DE 102007059470
(43) Veröffentlichungstag der Anmeldung: 01.09.2010
(73) Patentinhaber: *Acri. Tec GmbH, 16761 Hennigsdorf (DE)
(72) Erfinder: RITTER, Helmut, 42111 Wuppertal (DE); SCHMITZ, Daniel, 50677 Köln (DE)
(74) Vertreter: Lechner, Armin Anton
(86) Internationale Anmeldenummer: PCT/EP2008/066905
(87) Internationale Veröffentlichungsnummer: WO 2009/074521

(56) Entgegenhaltungen:
- DE-A1-102006 028 507
- US-A1- 2006 122 349

## Beschreibung

Die Erfindung betrifft eine ophthalmologische Zusammensetzung sowie ihre Verwendung, insbesondere als Augenimplantat (Intraokularlinse).

Es ist bekannt, dass die Netzhaut des Auges gegen phototoxische Einflüsse von Strahlung im Ultraviolettbereich (200 nm bis 400 nm) und violettem des sichtbaren Lichts Bereich (400 nm bis 440 nm) mit Hilfe von molekularen Absorbern geschützt werden kann. Derartige Absorber können im optischen Bereich zur Verwendung in Intraokularlinsen (IOL) vorgesehen sein. Auf dem Markt befindliche Intraokularlinsen absorbieren insbesondere im violetten Lichtbereich nur teilweise. Größenordnungsmäßig treten 25% bis 35% des phototoxischen Lichts mit einer Wellenlänge von 430 nm durch das herkömmliche Linsenmaterial hindurch.

Untersuchungen zeigen, dass der violette Lichtanteil eine entscheidende Rolle bei der Ausprägung einer Altersbedingten Makula-Degeneration (AMD) spielt. Diese beginnt durch Ablagerungen von so genannten Drusen, Stoffwechselendprodukten (Lipofuszinen), und kann in fortgeschrittenem Stadium in einen flächigen Zelltod (geographische Atrophie) des retinalen Pigmentepithels übergehen.

Andererseits ist für die Photorezeption, insbesondere für das Sehen bei verminderten Lichtverhältnissen (scotopic Vision), d.h. beim Dämmerungs- und Nachtsehen, die Durchlässigkeit des Linsenmaterials im blauen Lichtspektrum (etwa 450 nm bis 500 nm) von entschiedener Bedeutung. In diesem blauen Wellenlängenbereich soll so wenig Licht wie möglich absorbiert werden, um eine Beeinträchtigung des Dämmerungs- und Nachtsehens auszuschließen. Auf dem Markt befindliche IOL weisen in diesem Wellenlängenbereich (z.B. bei 475 nm) jedoch eine Transmission von nur etwa 70% bis 75% auf.

Aufgabe der Erfindung ist es daher, eine ophthalmologische Zusammensetzung der eingangs genannten Art zu schaffen, die ein hohes Maß an Photoprotektion bei gleichzeitiger maximaler Photorezeption gewährleistet. Diese Zusammensetzung muss also im wesentlichen den gesamten ultravioletten Spektralbereich und den violetten Lichtanteil des sichtbaren Spektrums absorbieren, dabei aber gleichzeitig die vollständige Transmission von blauem Licht, insbesondere des Wellenlängenbereichs zwischen 450 nm und 500 nm, ermöglichen. Diese Aufgabe wird erfindungsgemäß durch die Merkmale des Patentanspruchs 1 gelöst.

Die ophthalmologische Zusammensetzung der Erfindung beinhaltet einen UV-Absorber, der Strahlung im Wellenlängenbereich von etwa 200 nm bis 400 nm quantitativ absorbiert. Ferner beinhaltet die ophthalmologische Zusammensetzung einen Violett-Absorber, der violettes Licht der Wellenlängen von etwa 400 nm bis 430 nm absorbiert. Geeignete Chromophorgrundstrukturen des Violett-Absorbers sind N-alkoxyacrylierte bzw. N-alkoxymethacrylierte oder aber auch N,Ndialkoxyacrylierte bzw. N,N-dialkoxymethacrylierte Nitroaniline.

Als UV-Absorber beinhaltet die ophthalmologische Zusammensetzung ein biokompatibles UV-Lichtschutzmittel, wofür Cumarinderivate, die gegebenenfalls über Alkylspacer mit einer bzw. mehreren Acryl- oder Methacrylfunktionen ver-knüpft sind, verwendet werden.

Vorzugsweise ist die Zusammensetzung ausschließlich auf Acrylat- und/oder Methacrylatbasis aufgebaut.

Der Gegenstand der Erfindung wird in den Ansprüchen 1 bis 27 näher erläutert. UV-Absorber

Geeignete UV-Absorber der erfindungsgemäßen ophthalmologischen Zusammensetzung sind Verbindungen der folgenden Strukturen: 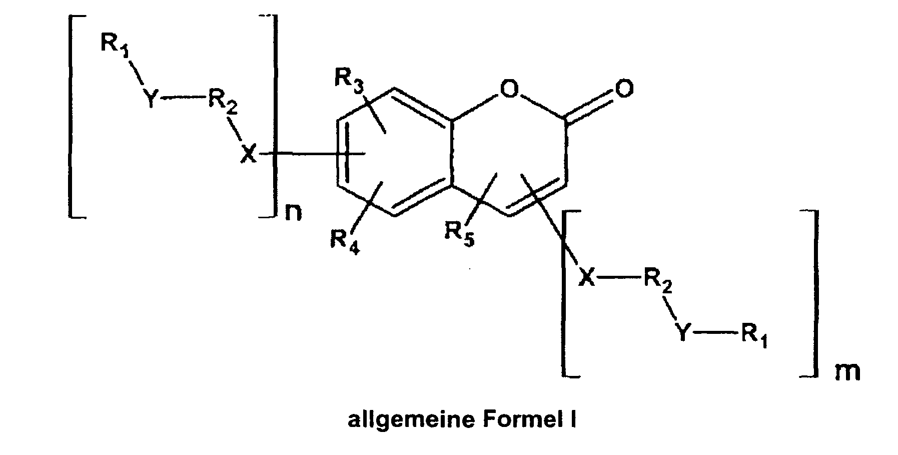
- n: = 0 bis 2
- m: = 0 oder 1 *wobei n*+*m* ≥ 1
- X: = O, NH, NR6
- Y: = O, NH, NR6
- R1: = Acryl- oder Methacrylrest

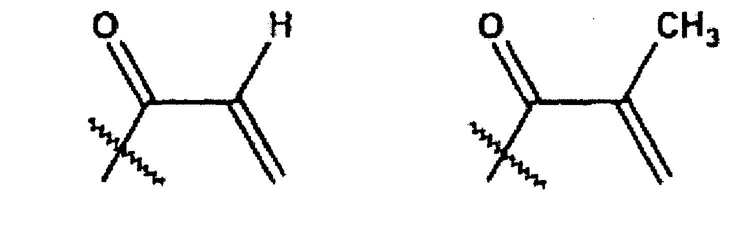

- R2: = organische Alkyl- oder Arylspacergruppe (oder Kombination aus beiden) mit bis zu 30 Atomen, ausgewählt aus: C, H, Si, O, N, P, S, Cl, Br, F
- R3,R5,R6: = H oder organische Alkyl- oder Arylgruppe (oder Kombination aus beiden) mit bis zu 30 Atomen, ausgewählt aus: C, H, Si, 0, N, P, S, Cl, Br, F
- R4: = *nur wenn n*=*0 oder 1*: H oder organische Alkyl- oder Arylgruppe (oder Kombinationen aus beiden) mit bis zu 30 Atomen, ausgewählt aus: C, H, Si, O, N, P, S, Cl, Br, F

Beispiele entsprechender Strukturen (alle Stereoisomere oder racemische Gemische sind beinhaltet) sind:

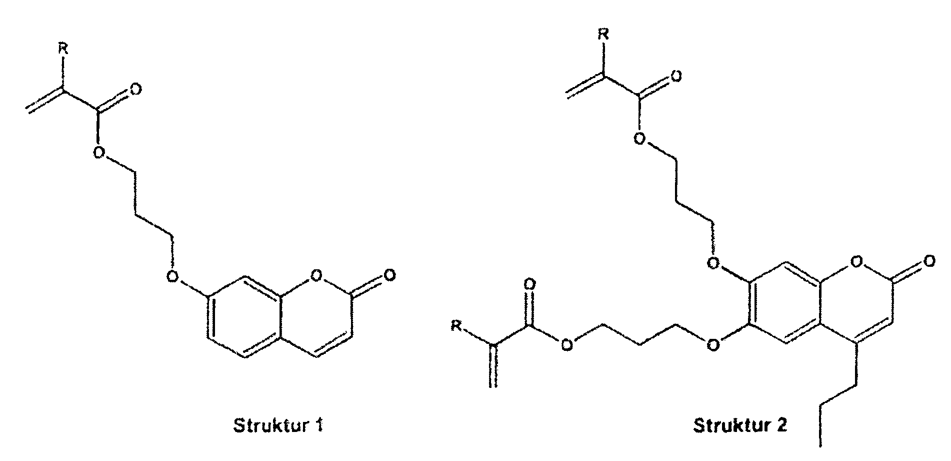

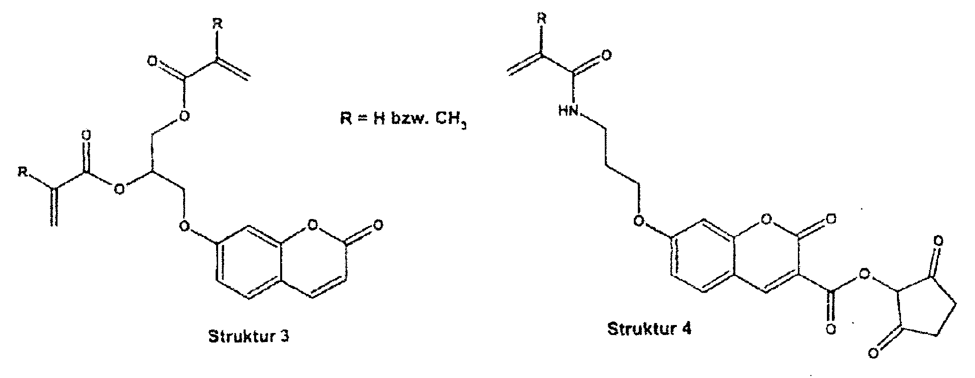

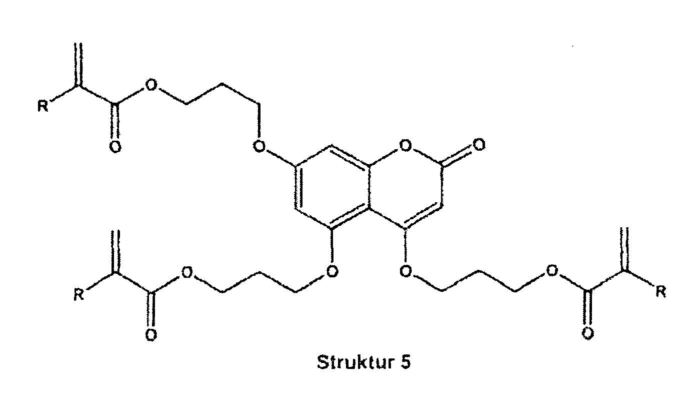

UV-Absorber, deren Grundstruktur auf den Strukturen 2, 3 und 4 basieren, haben den Vorteil, dass diese bedingt durch das Vorhandensein mehrerer polymerisierbarer Endgruppen einen quantitativen Einbau in das Linsenmaterial ermöglichen und darüber hinaus vernetzende Eigenschaften besitzen. Bei einer Linsenherstellung kann so im Idealfall auf die Zugabe eines zusätzlichen Vernetzers verzichtet werden.

Ein bevorzugter UV-Absorber der erfindungsgemäßen ophthalmologischen Zusammensetzung ist:

### Cumarin-7-propoxymethacrylat

mit der Struktur:

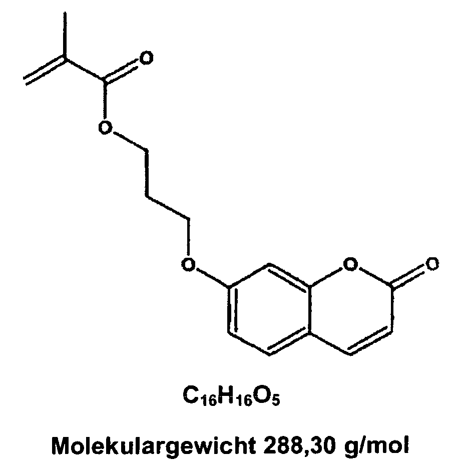

Die Herstellung dieser Verbindung erfolgt in zwei Schritten, wobei das 7-Hydroxycumarin kommerziell erhältlich ist:

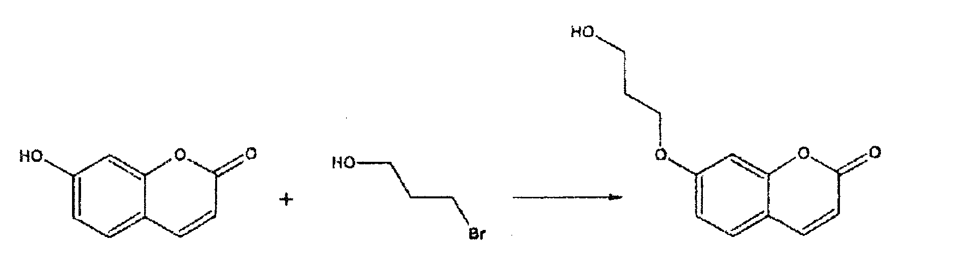

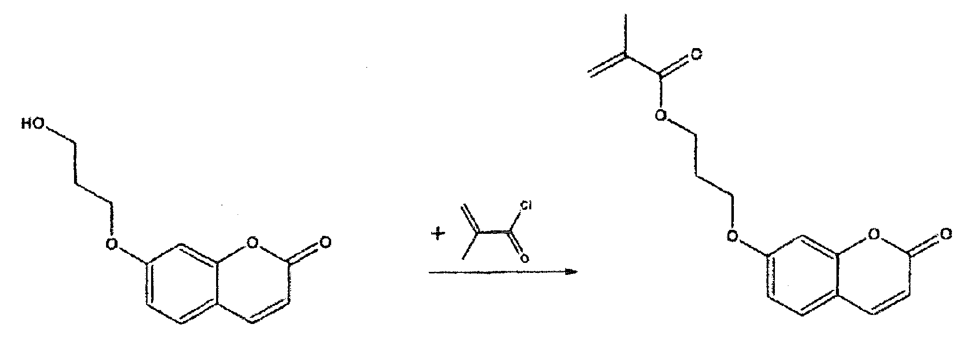

Weitere Ausführungsbeispiele für den UV-Absorber sind Verbindungen, bei denen ein Cumarin-Grundkörper über verschiedene Spacer mit einem oder mehreren Acryl- bzw. Methacryl-Resten verbunden ist. Diese besitzen folgende Struktur: 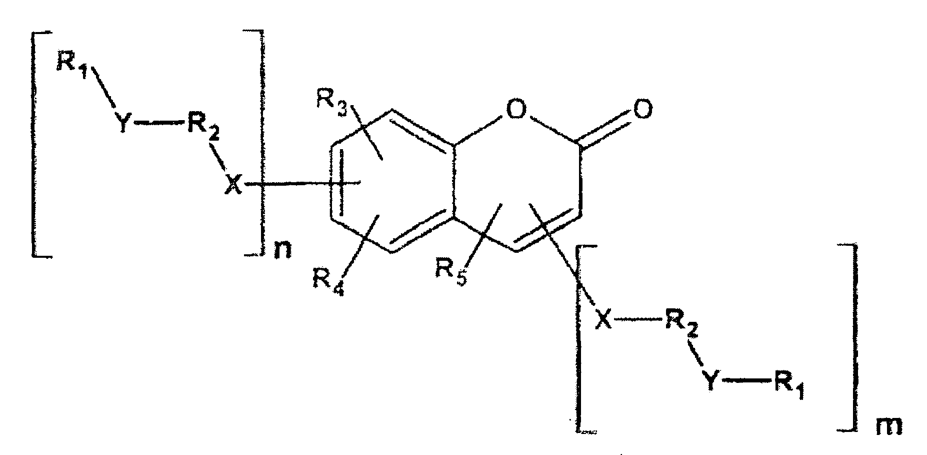 wobei
- R1 ein Acryl- bzw. Methacryl-Rest ist
- R2: organische verzweigte und unverzweigte Alkyl- oder Aryl-Substituenten (oder Kombinationen von beiden) mit bis zu 30 Atomen ausgewählt aus C, H, Si, O, N, P, S, F, Cl, Br
- R3, R4 und R5: H oder organische verzweigte und unverzweigte Alkyl-oder Aryl-Substituenten (oder Kombinationen von beiden) mit bis zu 30 Atomen ausgewählt aus C, H, Si, O, N, P, S, F, Cl, Br
- X und Y: O, S, NH, NR (R ist ein organischer verzweigter oder unverzweigter Alkyl- oder Aryl-Substituenten (oder Kombinationen von beiden) mit bis zu 30 Atomen ausgewählt aus C, H, Si, O, N, P, S, F, Cl, Br
- n = 0 bis 2 sowie m = 0 oder 1, wobei n+m immer größer gleich 1 ist

### Ausführungsbeispiele UV-Absorber

### Beispiel 1:

n = 2, m = 0, X = O, R₂ = C₃H₆, Y = O, R₁ = Acryl- bzw. Methacrylrest, R₃ = H, R₄ = H, R₅ = H in der allgemeinen Formel I.

Ein weiteres Ausführungsbeispiel für einen UV-Absorber im Sinne der erfindungsgemäßen ophthalmologischen Zusammensetzung ist Cumarin-6,7-dipropoxymethacrylat. Auch dieser kann auf einfachem synthetischem Wege analog dem Cumarin-7-propoxymethacrylat in einer 2-stufigen Reaktion dargestellt werden. Das dazu benötigte 6,7-Dihydroxycumarin ist ebenfalls kommerziell verfügbar. Auf diese Weise kann eine Verbindung hergestellt werden, bei der eine zusätzliche Methacrylat-Ankergruppe eingeführt wurde. Das Anbinden dieser zweiten Ankergruppe über einen Alkoxyspacer hat nur einen geringen Einfluss auf die spektralen Eigenschaften des Absorbers, ermöglicht jedoch diesen ebenfalls als Vernetzter bei der Herstellung des Linsenmaterials einzusetzen.

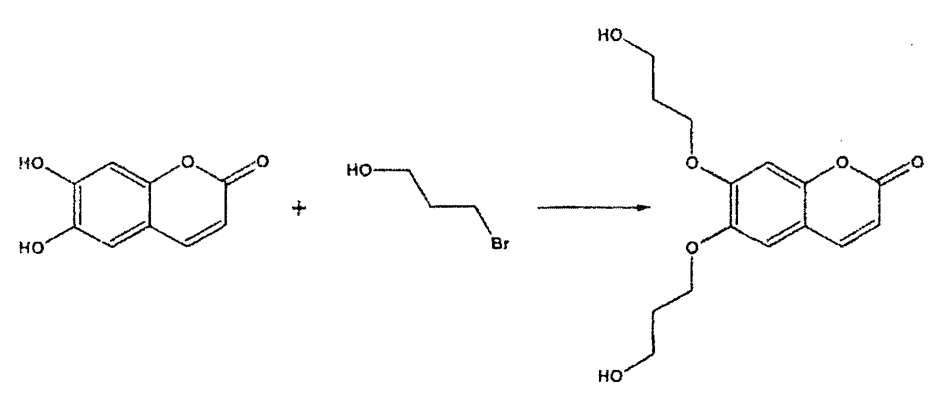

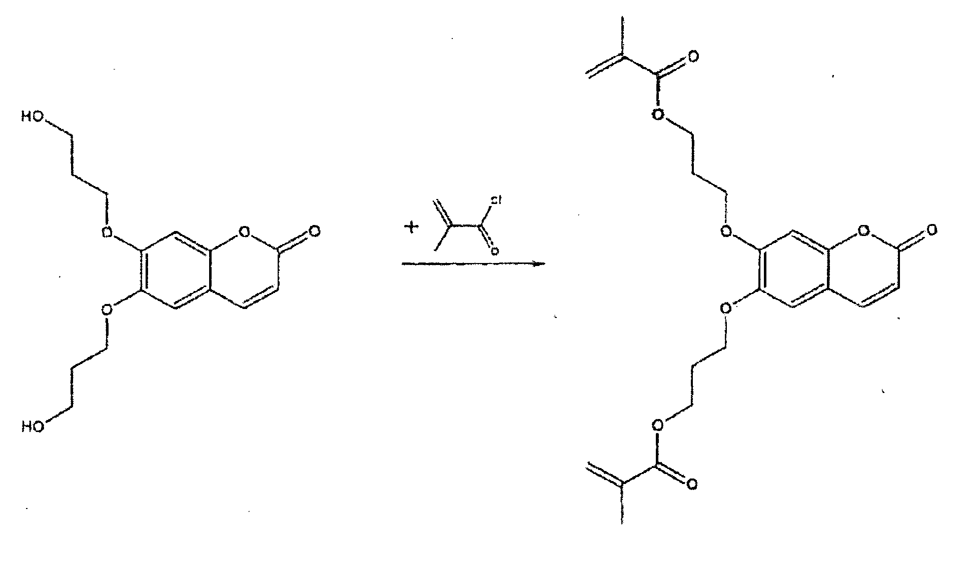

### Beispiel 2:

n = 1, m = 0, X = O, R₂ = -CH₂-CH(OR₁)CH₂-, Y = O, R1, = Acryl- bzw. Methacrylrest, R₃ = H, R₄ = H, R₅ = H der allgemeinen Formel I.

Eine weitere Möglichkeit einen UV-Absorber mit zwei Ankergruppen herzustellen ergibt sich aus der Verwendung eines verzweigten Dihydroxyhalogenids. Setzt man 7-Hydroxycumarin in einem ersten Schritt mit kommerziell erhältlichem 3-Brom-1,2-propandiol um und acryliert bzw. methacryliert das entstandene Alkoxydiol nachfolgend, erhält man einen weiteren bifunktionalen UV-Absorber.

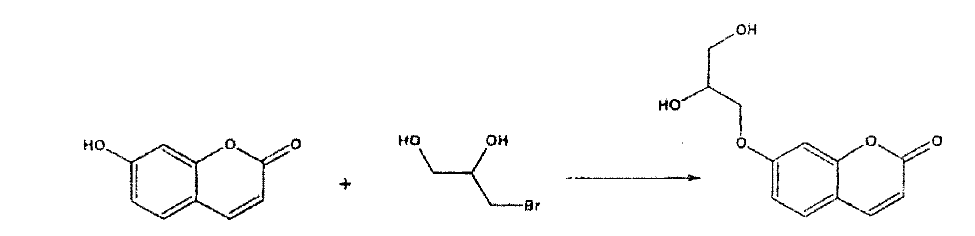

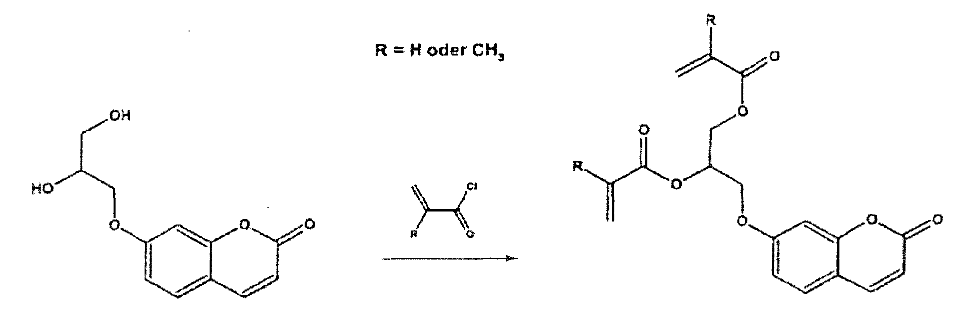

### Beispiel 3:

n = 1, m=0, X = O, R₂ = -CH₂-CH(OR1)CH₂-, Y = O, R₁ = Methacrylrest, R₃ = H, R₄ = H, R₅ = H der allgemeinen Formel I.

Setzt man 7-Hydroxycumarin nicht mit Acrylsäure bzw. Methacrylsäurechlorid, sondern mit kommerziell erhältlichem Methacrylsäureglycidylester um, so erhält man in einem einzigen Reaktionsschritt einen weiteren UV-Filter, in dem der Cumarin-Grundkörper durch eine aliphatische Kette vom Methacrylatrest getrennt ist. Durch nachfolgende Veresterung mit Methacryloylchlorid kann eine weitere Methacrylatfunktion an der sekundären Alkoholgruppe eingeführt werden.

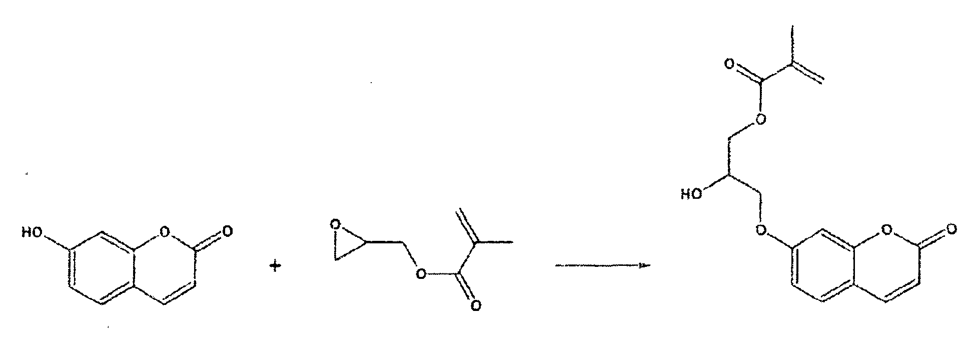

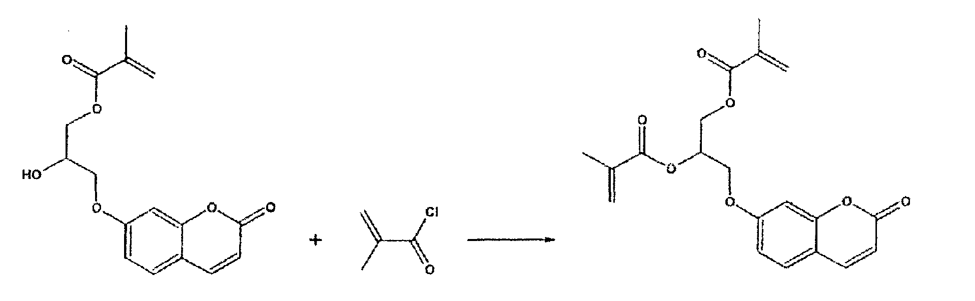

### Beispiel 4:

n = 1, m=0, X = O, R₂ = C₃H₆, Y = O, R₁ = Acryl- bzw. Methacrylrest, R₃ = H, R₄ = H, R₅ = C₃H₇ der allgemeinen Formel I.

Hier ist R₅ eine Propylgruppe, die einen schwachen induktiven Effekt (+I-Effekt) besitzt. Das Einbringen einer zusätzlichen Propylgruppe in den zuvor beschriebenen bevorzugten UV-Absorber ist synthetisch ohne Probleme zu bewältigen und verändert die spektralen Eigenschaften des Chromophors nur in geringem Maße. Setzt man bei der Synthese nicht 7-Hydroxycumarin sondern das ebenfalls kommerziell erhältliche 7-Hydroxy-4-propylcoumarin ein, erhält man nach der Methacrylierung ein Cumarinderivat, dass sich vom bevorzugten UV-Absorber um nur eine Propylseitenkette unterscheidet.

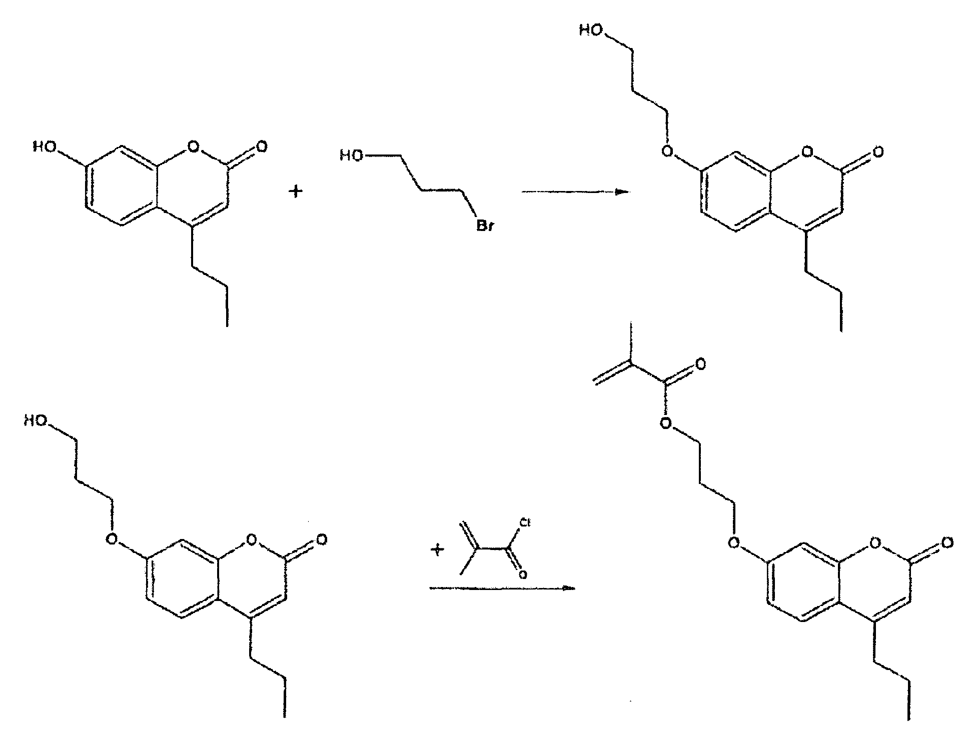

### Beispiel 5:

n = 2, m=1, X = O, R₂ = C₃H₆, Y = O, R₁ = Acryl- bzw. Methacrylrest, R₃ = H, R₄ = H, R₅ = H der allgemeinen Formel I.

Auch ein trifunktionaler UV-Absorber lässt sich auf einfachem synthetischem Wege herstellen. Ausgehend vom 4,5,7-Trihydroxycumarin erhält man nach der Alkoxylierung mit 3-Brom-1-propanol und nachfolgender Acrylierung bzw. Methacrylierung einen UV-Absorber mit drei Ankergruppen.

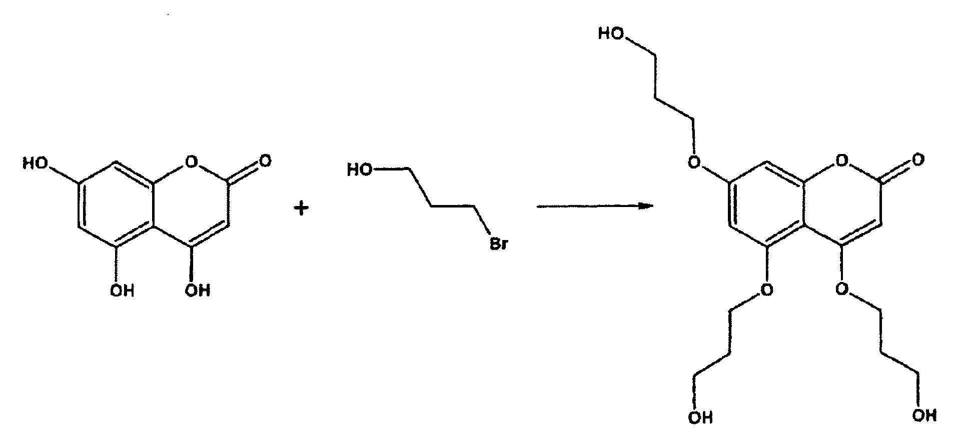

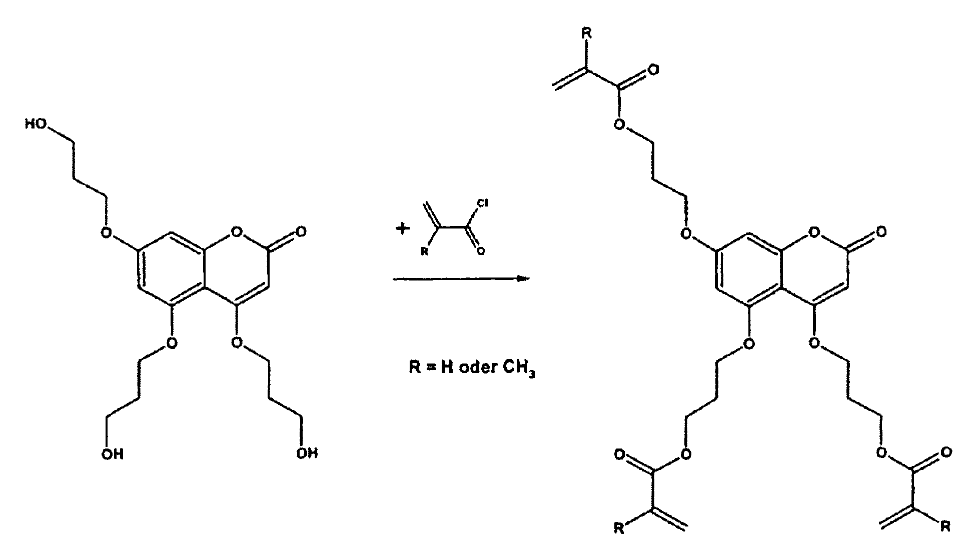

### Violett-Absorber

Geeignete Violett-Absorber der erfindungsgemäßen ophthalmologischen Zusammensetzung sind Verbindungen der folgenden Strukturen: 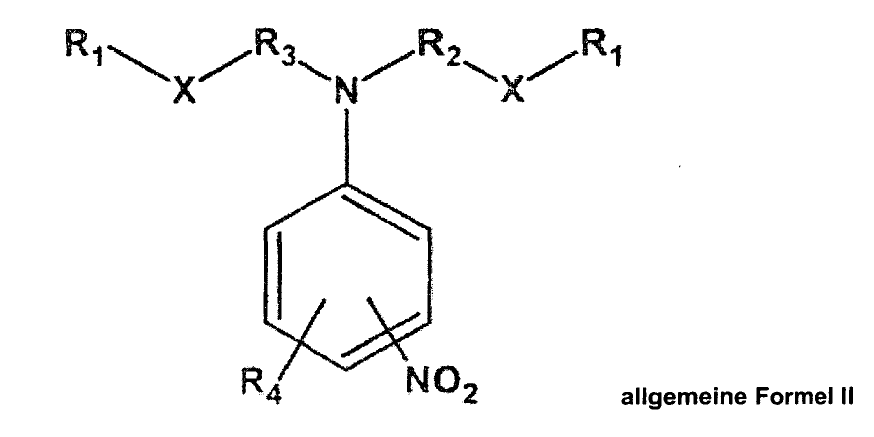
- X: = O, S, NH, NR (R ist ein organischer verzweigter oder unverzweigter Alkyl- oder Aryl-Substituent (oder Kombinationen von beiden) mit bis zu 30 Ato- men ausgewählt aus C, H, Si, O, N, P, S, F, Cl, Br)
- R₁: = Acryl- oder Methacrylrest

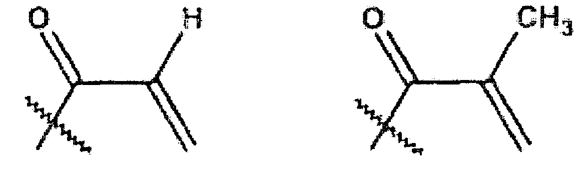

- R₂: = Organische verzweigte und unverzweigte Alkyl- oder Arylspacergruppe (oder Kombination aus beiden) mit bis zu 30 Atomen, ausgewählt aus: C, H, Si, O, N, P, S, Cl, Br, F
- R₃: = organischer verzweigter oder unverzweigter Alkyl- oder Aryl-Substituent (oder Kombination aus beiden) mit bis zu 30 Atomen, ausgewählt aus: C, H, Si, O, N, P, S, Cl, Br, F

- R₄: = H oder organischer verzweigter oder unverzweigter Alkyl- oder Aryl- Substituent (oder Kombination aus beiden) mit bis zu 30 Atomen, ausge- wählt aus: C, H, Si, O, N, P, S, Cl, Br, F oder weitere Nitrogruppe, Alko- xygruppe oder Nitrilgruppe

Beispiele entsprechender Strukturen (alle Stereoisomere oder racemische Gemische sind beinhaltet) sind:

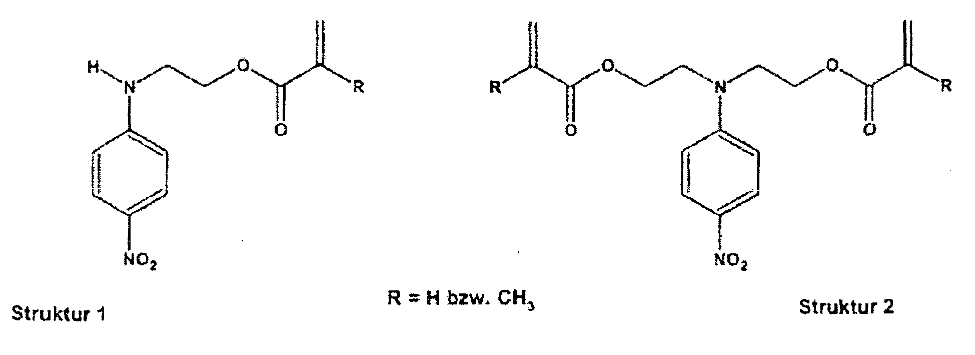

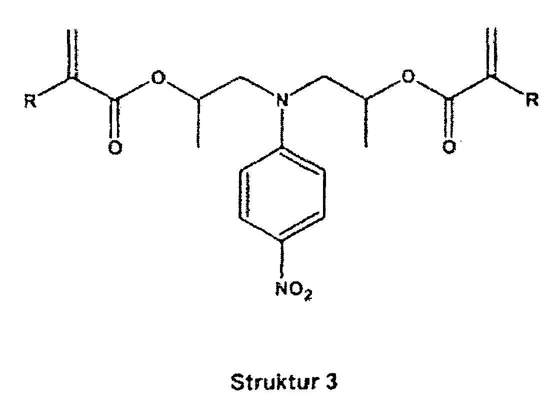

Ferner sind geeignete Violett-Absorber Stereoisomere oder racemische Gemische von Verbindungen folgender Strukturen: 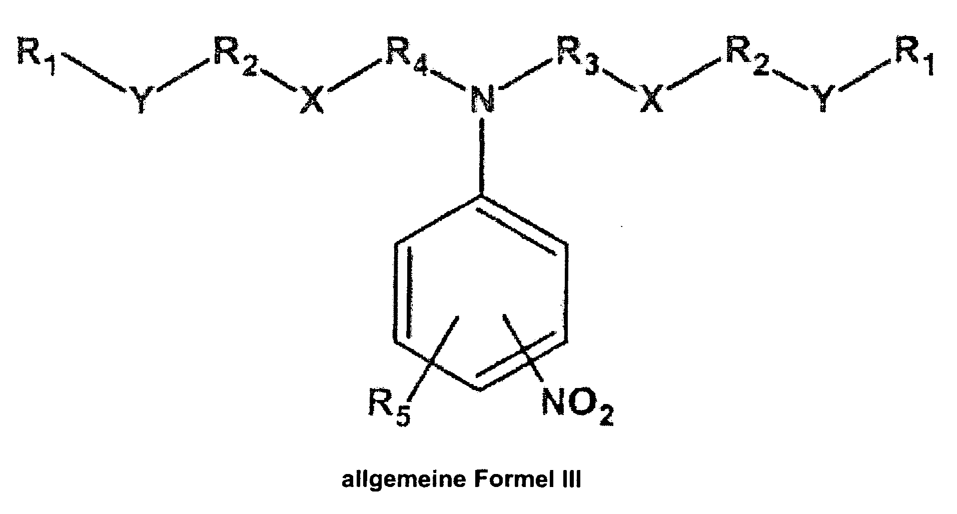
- X: = O, S, NH, NR (R ist ein organischer verzweigter oder unverzweigter Alkyl- oder Aryl-Substituenten (oder Kombinationen von beiden) mit bis zu 30 A- tomen ausgewählt aus C, H, Si, O, N, P, S, F, Cl, Br)
- Y: = O, S, NH, NR (R ist ein organischer verzweigter oder unverzweigter Alkyl- oder Aryl-Substituent (oder Kombinationen von beiden) mit bis zu 30 Ato- men ausgewählt aus C, H, Si, O, N, P, S, F, Cl, Br)
- R₁: = Acryl- oder Methacrylrest

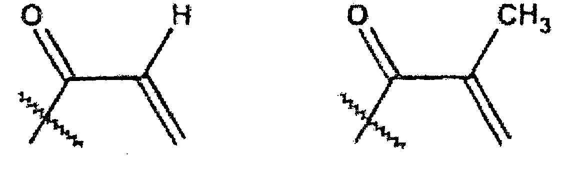

- R₂: = Organische verzweigte oder unverzweigte Alkyl- oder Arylspacergruppe (oder Kombination aus beiden) mit bis zu 30 Atomen, ausgewählt aus: C, H, Si, O, N, P, S, Cl, Br, F
- R₃: = Organische verzweigte oder unverzweigte Alkyl- oder Arylspacergruppe (oder Kombination aus beiden) mit bis zu 30 Atomen, ausgewählt aus: C, H, Si, O, N, P, S, Cl, Br, F
- R₄: = organischer verzweigter oder unverzweigter Alkyl- oder Aryl-Substituent (oder Kombination aus beiden) mit bis zu 30 Atomen, ausgewählt aus: C, H, Si, O, N, P, S, Cl, Br, F
- R₅: = H oder organischer verzweigter oder unverzweigter Alkyl- oder Aryl- Substituent mit bis zu 30 Atomen, ausgewählt aus: C, H, Si, O, N, P, S, Cl, Br, F oder weitere Nitrogruppe, Alkoxygruppe oder Nitrilgruppe

Beispiele entsprechender Strukturen (alle Stereoisomere oder racemische Gemische sind beinhaltet) sind: 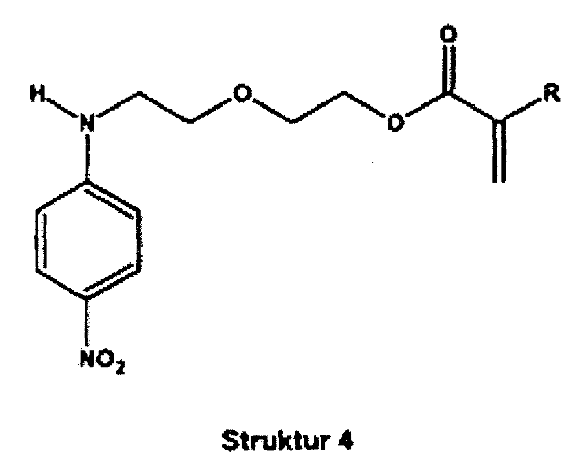 **R=H bzw. CH₃**

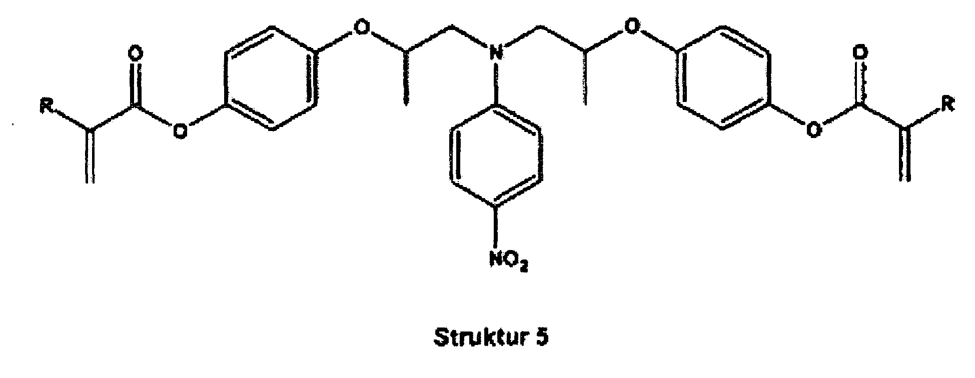

Ein bevorzugter Farbstoff für den Violett-Absorber der erfindungsgemäßen ophthalmologischen Zusammensetzung ist:

### N,N-Di-2'-ethylmethacrylat-4-nitroanilin

mit der Struktur:

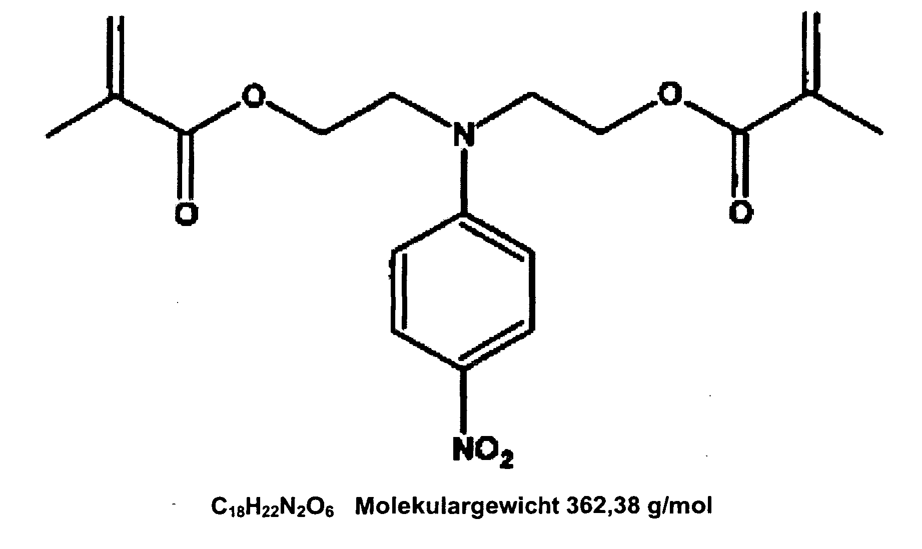

Die Herstellung dieser Verbindung erfolgt in zwei Schritten (gemäß Offenlegungsschrift EP 0321891 A2), wobei beide Edukte, sowohl das 4-Fluornitrobenzol, als auch das Diethanolamin kommerziell erhältlich sind:

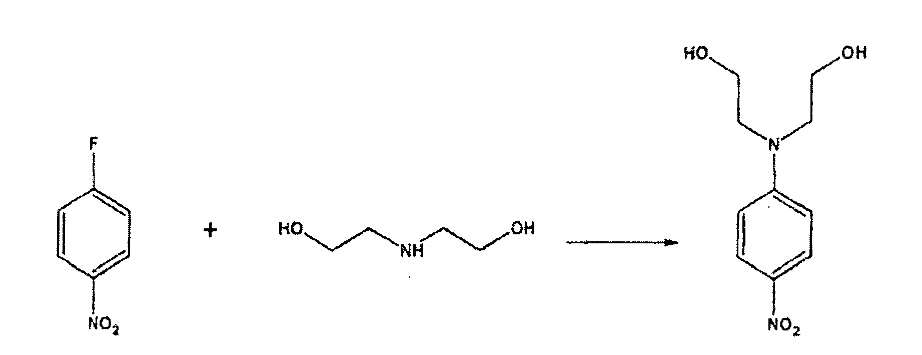

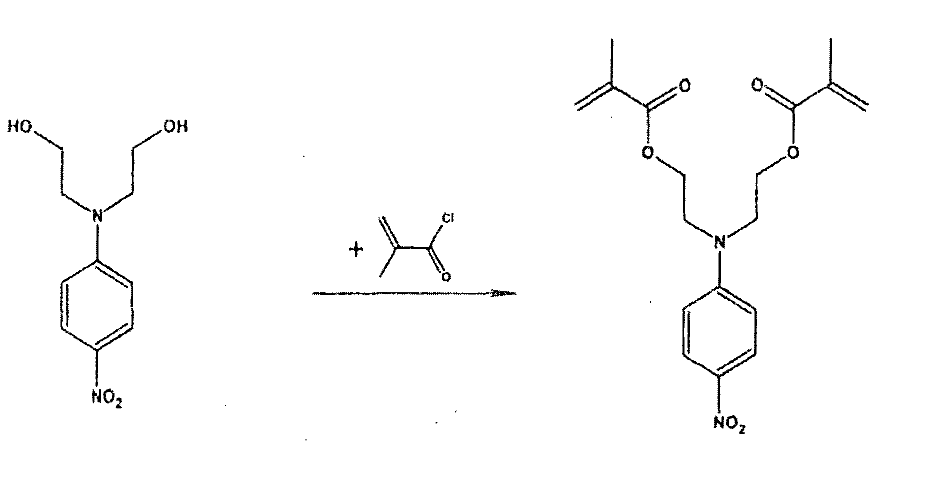

Die Methacryl-Reste dienen zur kovalenten Einbindung des Violett-Filters in ein Trägermaterial, insbesondere Linsenmaterial auf Acrylat-Basis. Aufgrund der Bifunktionalität verläuft der Einbau quantitativ und somit deutlich effektiver als bei den am Markt erhältlichen monofunktionalen Violett-Filtern.

Weitere Ausführungsbeispiele für den Violett-Absorber sind ebenfalls Verbindungen, bei denen ein Nitroanilin Grundkörper über verschiedene Spacer mit einem oder mehreren Acryl- bzw. Methacryl-Resten verbunden ist.

### Ausführungsbeispiele Violett-Absorber

### Beispiel 1:

R₂ und R₃ = -CH₂-CH(CH₃)- X = O, R₁ = Acryl- oder Methacrylrest, R₄ = H der allgemeinen Formel II.

Ein weiteres Ausführungsbeispiel für einen gelben Chromophor/Violett-Filter ist das N,N-Di-2'-isopropylmethacrylat-4-nitroanilin. Auch dieses kann auf einfachem synthetischem Wege analog dem Diethylmethacrylat-4-nitroanilin in einer 2-stufigen Reaktion hergestellt werden. Das dazu benötigte Diisopropanolamin ist ebenfalls kommerziell verfügbar. Auf diese Weise kann eine Verbindung hergestellt werden, die sich vom bevorzugten Filter um nur jeweils eine CH₃-Gruppe in der Seitenkette unterscheidet. Durch den positiven induktiven Effekt der Methylgruppen absorbiert dieser Chromophor geringfügig zu längeren Wellenlängen verschoben.

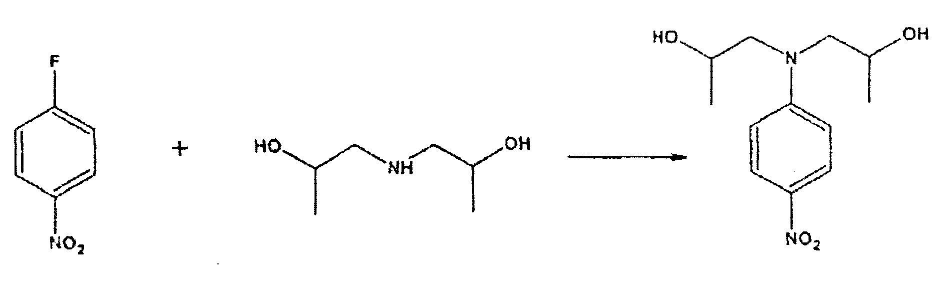

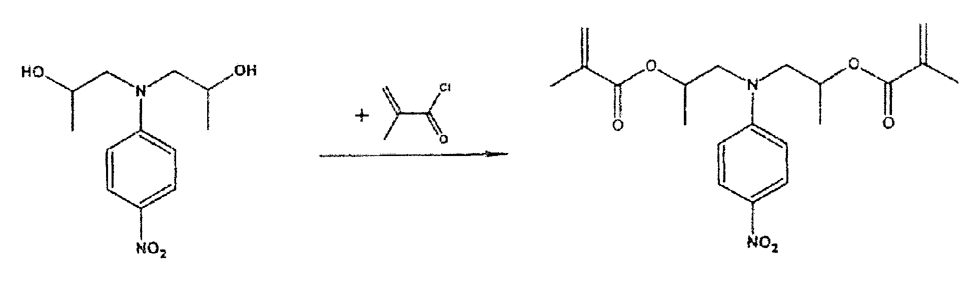

### Beispiel 2:

R₂ und R₃ = C₂H₄, X = NR, R₁ = Acryl- oder Methacrylrest, R₄ = H der allgemeinen Formel II.

Bei diesem Beispiel wird N,N-Dihydroxyethyl-4-nitroanilin durch eine einfache synthetische Methode in das Diamino-Derivat umgesetzt. Dieses Diamin kann durch eine Reaktion mit Acrylsäurechlorid in das Diamid umgewandelt werden. Die Struktur des Chromophors bleibt unverändert und ist durch zwei Methyleneinheiten vom Acryl-Amid getrennt.

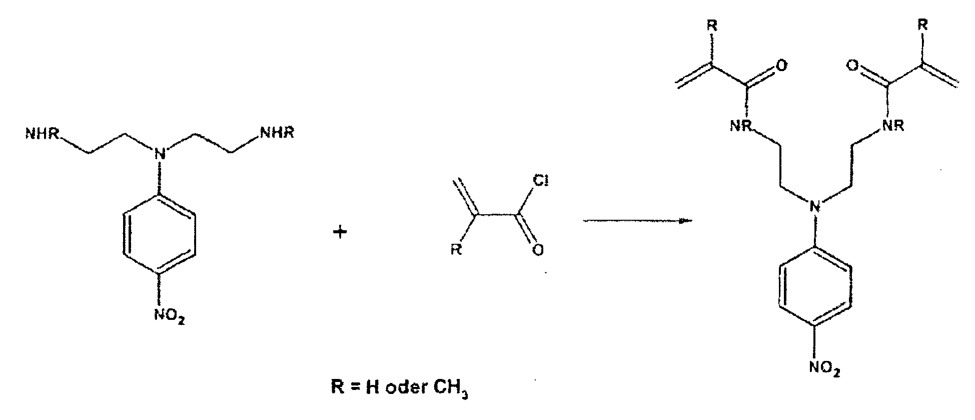

### Beispiel 3:

R₃ und R₄=C₂H₄, X = O, R₂ = -CH₂-CH(OH)CH₂-, Y = O, R₁ = Acryl- oder Methacrylrest, R₅ = H der allgemeinen Formel III.

Setzt man N,N-Dihydroxyethyl-4-nitroanilin nicht mit Acrylsäure bzw. Methacrylsäurechlorid, sondern mit kommerziell erhältlichem Methacrylsäureglycidylester um, so erhält man in einem einzigen Reaktionsschritt einen weiteren Violett-Filter, in dem der Chromophor durch aliphatische Ketten von den Methacrylatresten getrennt ist.

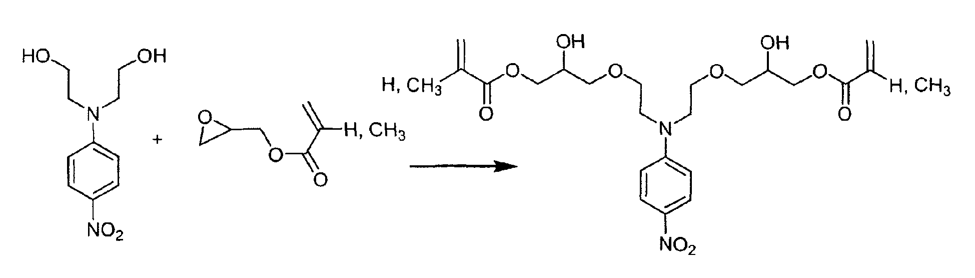

### Beispiel 4:

R₂ und R₃ = C₂H₄, X = O, R₁ = Acryl- oder Methacrylrest, R₄ = CH₃ der allgemeinen Formel II.

Hier ist R₄ eine Methylgruppe, die einen schwachen induktiven Effekt (+I-Effekt) besitzt. Das Einbringen einer zusätzlichen Methylgruppe in den zuvor beschriebenen bevorzugten Violett-Filter ist synthetisch ohne Probleme zu bewältigen und verändert die spektralen Eigenschaften des Chromophors nur in geringem Maße. Setzt man Diethanolamin nicht mit 4-Fluornitrobenzen, sondern mit dem ebenfalls kommerziell erhältlichen 2-Fluor-5-nitrotoluol um, so wird ein Nitroanilin erzeugt, das sich vom bevorzugten Violett-Absorber um gerade eine zusätzliche Methylgruppe am Anilin-Ring unterscheidet. Durch Veresterung mit Acryloylchlorid oder Methacryloylchlorid wird so ein weiterer Chromophor mit den gewünschten spektralen Eigenschaften erhalten.

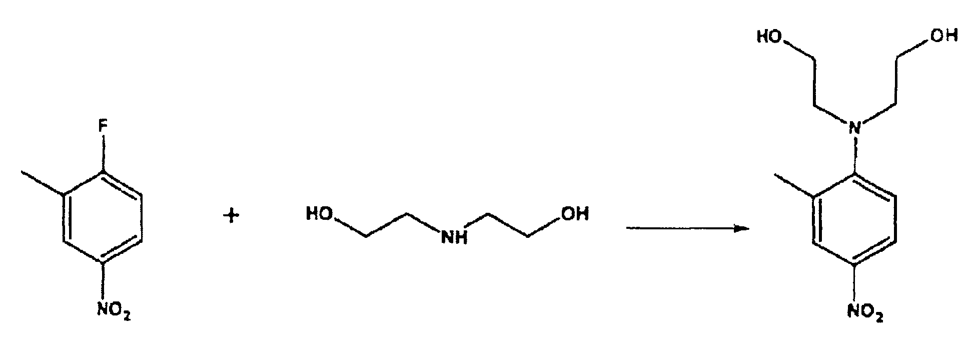

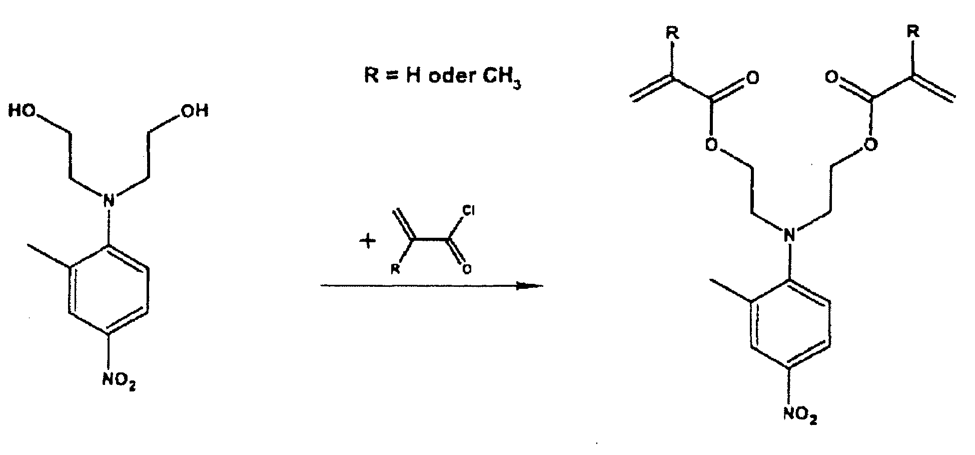

Als biokompatibles Trägermaterial sind Acrylate, insbesondere mit einem Wassergehalt von 1 % bis 30%, für die ophthalmologische Zusammensetzung geeignet. In dem Copolymer bzw. in diesem Trägermaterial sind der UV-Absorber bzw. der Violett-Absorber kovalent eingebunden. Der UV-Absorber ist vorzugsweise in einem Konzentrationsbereich von 0,5 % bis 1,0 % enthalten. Wenn die ophthalmologische Zusammensetzung für eine IOL verwendet wird, hängt die jeweilige Konzentration des UV-Absorbers vom jeweiligen Scheitelbrechwert (Dioptrie) der Linse ab. Der Violett-Absorber ist ebenfalls kovalent im Acrylat-Trägermaterial bzw. im Copolymer gebunden. Er kann in einem Konzentrationsbereich von 0,03% bis 0,16% vorliegen. Auch hier hängt bei der Verwendung der ophthalmologischen Zusammensetzung für eine IOL die Konzentration des Violett-Absorbers direkt von der Dioptrie der Linse ab.

Die Gefahr des Auswaschens der Absorber aus der Trägermatrix besteht nicht, da sowohl der erfindungsgemäße UV-Absorber als auch der Violett-Filter bedingt dadurch, dass sie zwei polymerisationsfähige Endgruppen tragen, quantitativ in das Linsenmaterial einbauen.

Geeignete biokompatible Trägermaterialien für den UV-Absorber bzw. den Violett-Absorber sind beispielsweise Hydroxyethylmethacrylat (HEMA), Methylmethacrylat (MMA), Ethoxyethylmethacrylat (EOEMA), Ethoxyethoxyethylacrylat (EEEA), Tetrahydrofufurylmethacrylat (THFMA), Tetrahydrofufurylacrylat (THFA), 2-Hydroxypropylmethacrylat (HPMA), 2-Hydroxypropylacrylat (HPA), 2-Hydroxyethylacrylamid, 2-Hydroxyethylmethacrylamid, Methoxyethylmethacrylat (MOEMA) und Methoxyethylacrylat (MOEA). Aus den zuvor genannten Stoffen können Copolymerisate, evtl. unter Verwendung eines Quervemetzers, hergestellt und als Trägermaterial verwendet werden. Die prozentuale Zusammensetzung der Monomere ist in einem weiten Bereich variabel. Die Trägermaterialien können hydrophil mit einem Wassergehalt von beispielsweise 1% bis 30% oder hydrophob eingestellt werden. Begrenzender Faktor bei hydrophoben, wasserfreien Polymeren ist der Glaspunkt. Dieser kann im Bereich zwischen 0°C und 11°C liegen. Darüber hinaus ist wichtig, dass hydrophile Polymere nach der Quellung eine ausreichende Flexibilität aufweisen.

Ausführungsbeispiele der ophthalmologischen Zusammensetzung sind folgende mit quantitativen Zusammensetzungen in Gewichts %.

### Ausführungsbeispiel Trägermaterialien

### Beispiel 1 (hydrophob)

EOEMA (Ethoxyethylmethacrylat) 85 - 97 Gew.-%
MMA (Methylmethacrylat) 0-15 Gew.-%
EEEA (Ethoxyethoxyethylacrylat) 0 - 5 Gew.-%
EGDMA (Ethylenglykoldimethacrylat) 0 - 0,7 Gew.-%
UV-Absorber 0,1 -1,0 Gew.-%
Violett-Absorber 0,03-0,16 Gew.-%

### Beispiel 2 (hydrophil)

HEMA (Hydroxyethylmethacrylat) 50 - 85 Gew.-%
EOEMA (Ethoxyethylmethacrylat) 30 - 40 Gew.-%
THFMA (Tetrahydrofufurylmethacrylat) 5-20 Gew.-%
EGDMA (Ethylenglykoldimethacylat) 0 - 0,7 Gew.-%
UV-Absorber 0,1-1,0 Gew.-%
Violett-Absorber 0,03 - 0,16 Gew.-%

Zur Synthese der jeweiligen Linsenmaterialien werden in ein Becherglas nacheinander zunächst die Monomere eingewogen und solange gerührt, bis eine homogene Lösung entstanden ist. Danach wird zunächst der Vernetzer und nachfolgend der Violett- sowie der UV-Absorber zugegeben. Unter leichtem Erwärmen wird wiederum so lange nachgerührt bis eine homogene Lösung erhalten wird.

Die sich jeweils ergebende Mischung wird mit einem geeigneten Initiator versetzt und in die Polymerisationsformen überführt (z.B. Näpfe, Stangen- oder Plattformen). Die Polymerisation wird durch Erhitzen eingeleitet (60°C für 12-16 h). Nach dem Erkalten werden die Polymerisate entnommen, ggf. im Trockenschrank nachgehärtet und durch Drehen und Fräsen auf die gewünschte Rohlingsgröße gebracht (z.B. 3 mm Stärke, 12,7 mm Durchmesser).

Transmissionsmessungen zeigen, dass mit Hilfe der erfindungsgemäßen ophthalmologischen Zusammensetzung nicht nur der UV-Anteil (<400 nm), sondern auch der gesamte violette Lichtanteil (400 nm bis 430 nm) absorbiert wird. Auf dem Markt befindliche ophthalmologische Zusammensetzungen weisen im violetten Bereich eine hohe Lichtdurchlässigkeit mit bis zu einem Drittel Transmission auf. Die erfindungsgemäße Zusammensetzung zeigt bei 430 nm lediglich eine Transmission von unter 3 %.

Im blauen Lichtbereich hat die erfindungsgemäße Zusammensetzung beispielsweise bei 460 nm eine Lichtdurchlässigkeit von über 70 %, während die bekannten Linsen hier nur 50 -60 % Durchlässigkeit aufweisen.

Die ophthalmologische Zusammensetzung eignet sich insbesondere für Sehhilfen, wie Brillen, Kontaktlinsen und Augenimplantate. Insbesondere eignet sich die erfindungsgemäße ophthalmologische Zusammensetzung für Intraokularlinsen.

## Patentansprüche

1. Ophthalmologische Zusammensetzung, welche auf Acrylat- und/oder Methacrylatbasis aufgebaut ist und welche einen UV-Absorber aufweist, bei dem ein oder mehrere Acrylsäure bzw. Methacrylsäure-Einheiten über einen Alkylspacer an eine substituierte oder unsubstituierte Cumarin-Grundstruktur gebunden sind, sowie einen Violett-Absorber (Gelbfarbstoff) auf Basis eines N-alkoxyacrylierten bzw. N-alkoxymethacrylierten oder auf Basis eines N,N-dialkoxyacrylieten bzw. N,N-dialkoxymethacrylierten substituierten oder unsubstituierten Nitroanilins enthält.

2. Ophthalmologische Zusammensetzung nach Anspruch 1, bei welcher der UV-Absorber ein Stereoisomer oder racemisches Gemisch folgender Struktur ist: 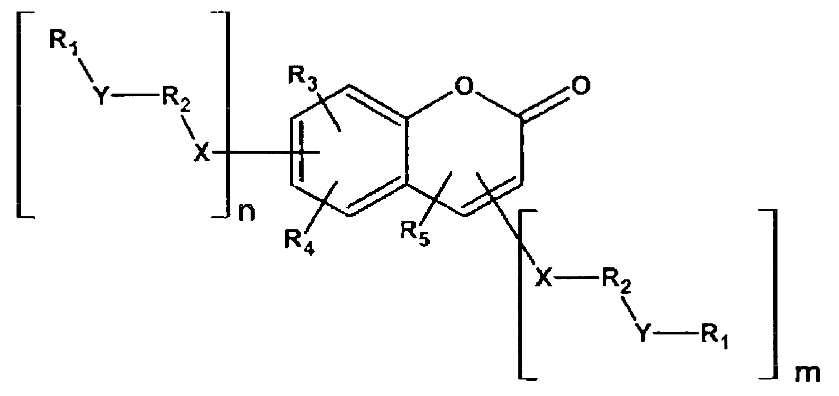 wobei
• R1 ein Acryl- bzw. Methacryl-Rest ist
• R2: organische verzweigte und unverzweigte Alkyl- oder Aryl-Substituenten (oder Kombinationen von beiden) mit bis zu 30 Atomen ausgewählt aus C, H, Si, O, N, P, S, F, Cl, Br
• R3, R4 und R5: H oder organische verzweigte und unverzweigte Alkyl- oder Aryl-Substituenten (oder Kombinationen von beiden) mit bis zu 30 Atomen ausgewählt aus C, H, Si, O, N, P, S, F, Cl, Br
• X und Y: 0, S, NH, NR (R ist ein organischer verzweigter oder unverzweigter Alkyl- oder Aryl-Substituent (oder Kombinationen von beiden) mit bis zu 30 Atomen ausgewählt aus C, H, Si, O, N, P, S, F, Cl, Br
• n = 0 bis 2 sowie m = 0 oder 1 wobei n+m immer größer gleich 1 ist.

3. Ophthalmologische Zusammensetzung nach Anspruch 2, **dadurch gekennzeichnet, dass** n = 1, m=0, X = O, R₂ = C₃H₆, Y = O, R₁ = Acryl- bzw. Methacrylrest, R₃ = H, R₄ = H, R₅ = H mit der Struktur: 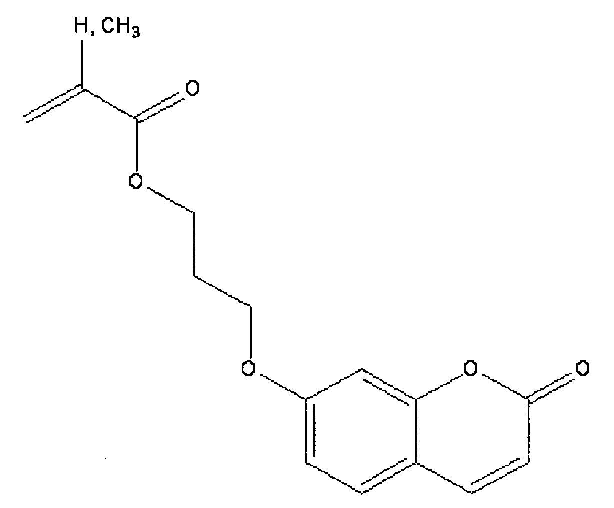 oder
n = 2, m=0, X = O, R₂ = C₃H₆, Y = O, R₁ = Acryl- bzw. Methacrylrest, R₃ = H, R₄ = H, R₅ = H mit der Struktur: 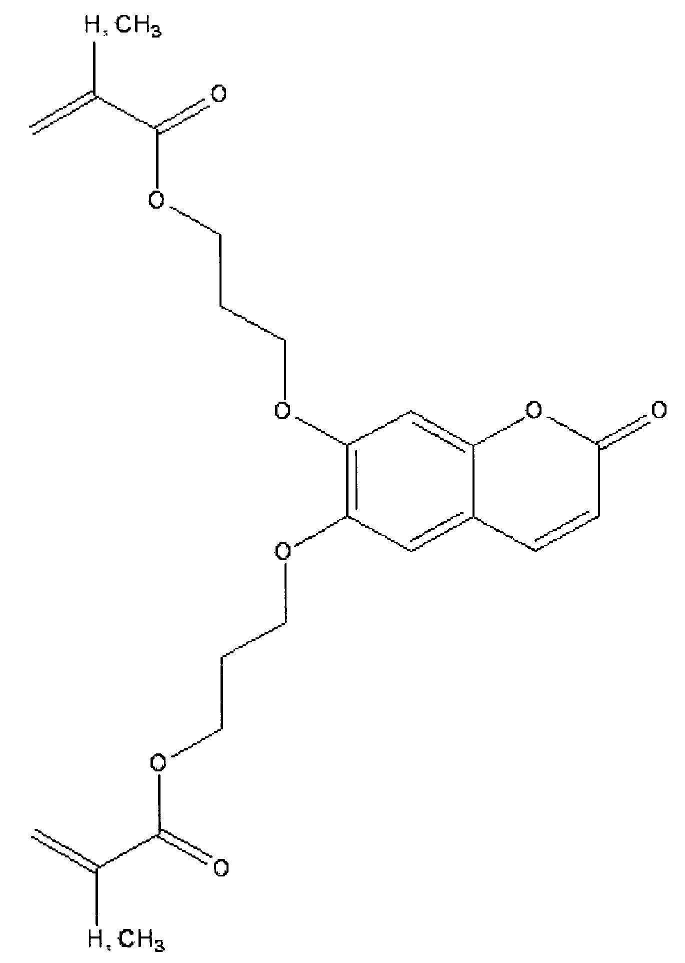 oder
n = 1, m=0, X = O, R₂ = -CH₂-CH(OR₁)CH₂-, Y = O, R₁ = Acryl- bzw. Methacrylrest, R₃ = H, R₄ = H, R₅ = H mit der Struktur: 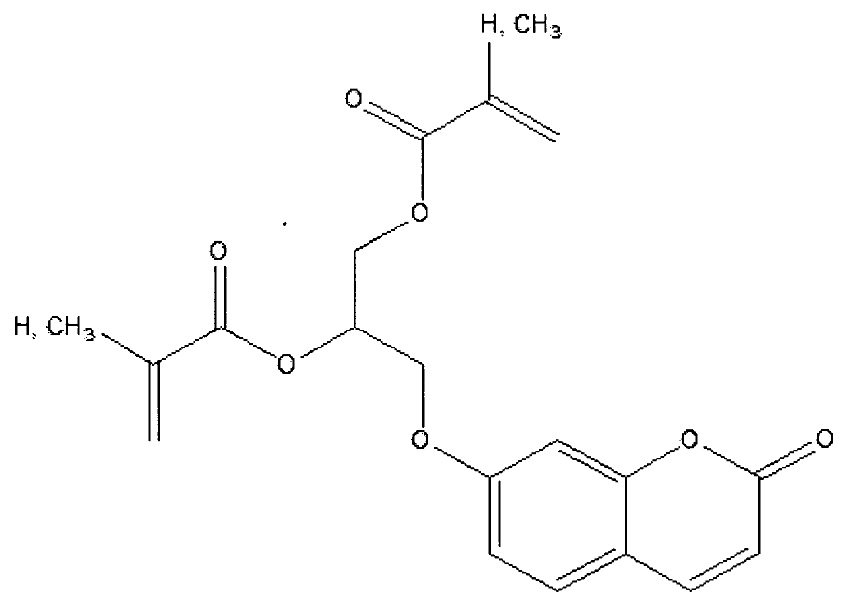 ist.

4. Ophthalmologische Zusammensetzung nach Anspruch 2, **dadurch gekennzeichnet, dass** n = 1, m = 0, X = O, R₂ = -CH₂-CH(OH)CH₂-, Y = O, R₁ = Acryl- bzw. Methacrylrest, R₃ = H, R₄ = H, R₅ = H mit der Struktur: 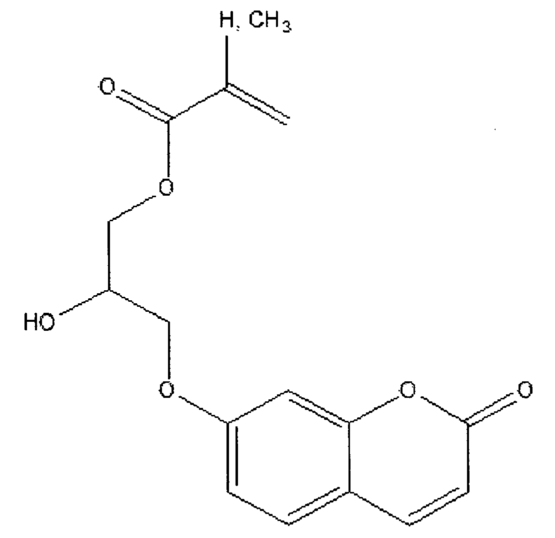 oder n = 1, m = 0, X = O, R₂ = C₃H₆, Y = O, R₁ = Acryl- bzw. Methacrylrest, R₃ = H, R₄ = H, R₅ = C₃H₇ mit der Struktur: 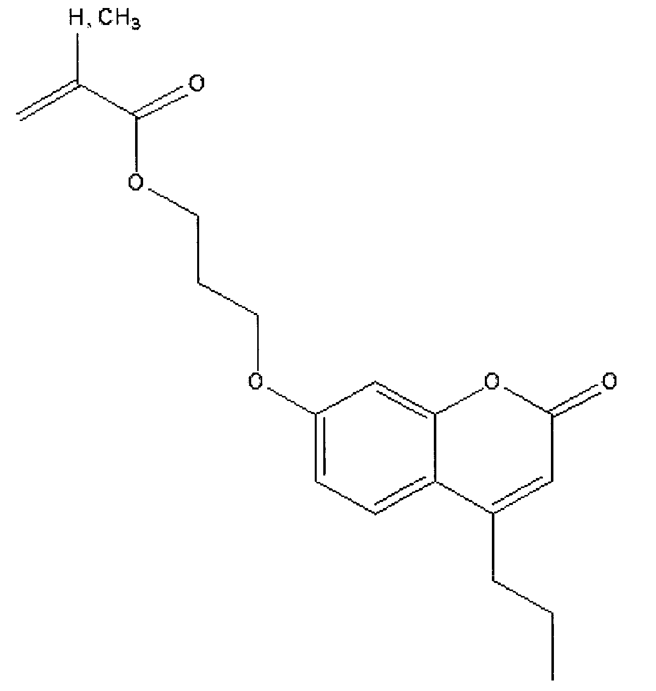 oder
n = 2, m = 1, X = O, R₂ = C₃H₆, Y = O, R₁ = Acryl- bzw. Methacrylrest, R₃ = H, R₄ = H, R₅ = H mit der Struktur: 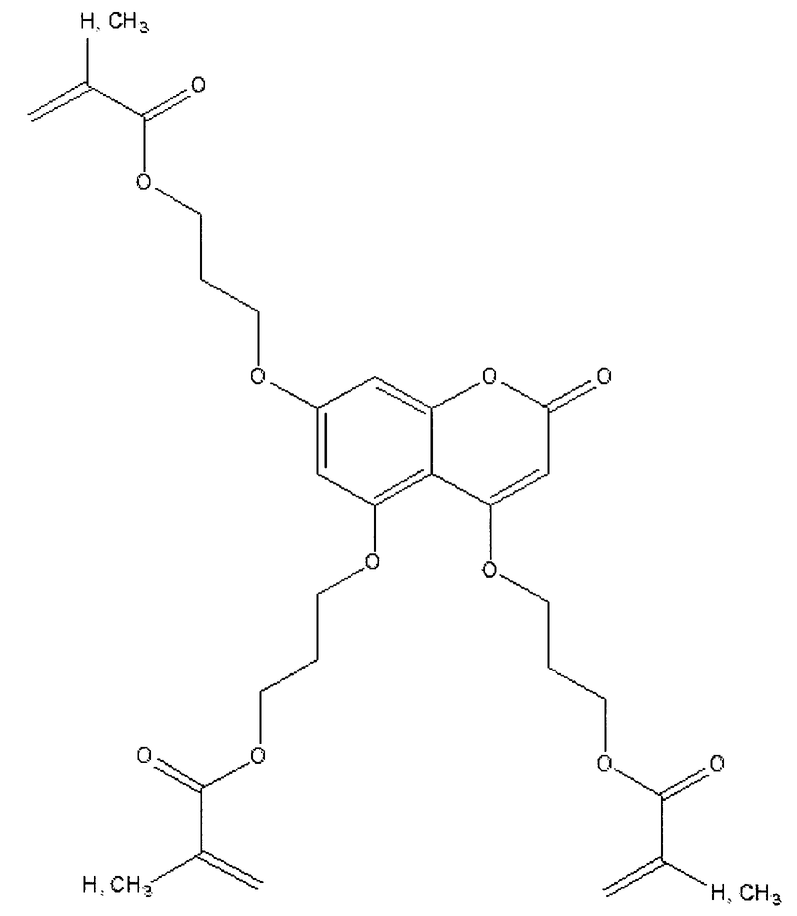 ist.

5. Ophthalmologische Zusammensetzung nach einem der Ansprüche 1 bis 4, **dadurch gekennzeichnet, dass** der Violett-Absorber ein Stereoisomer oder ein racemisches Gemisch von Farbstoffen folgender Struktur ist: 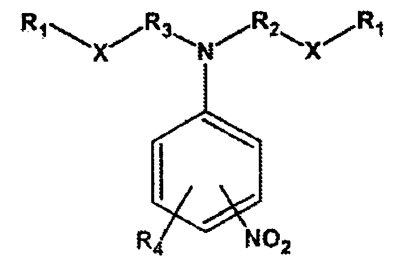 wobei
• R₁ ein Acryl- bzw. Methacryl-Rest ist
• R₂: organische verzweigte und unverzweigte Alkyl- oder Aryl-spacergruppe (oder Kombinationen von beiden) mit bis zu 30 Atomen ausgewählt aus C, H, Si, O, N, P, S, F, Cl, Br
• R₃: organischer verzweigter und unverzweigter Alkyl- oder Aryl-Substituent (oder Kombinationen von beiden) mit bis zu 30 Atomen ausgewählt aus C, H, Si, O, N, P, S, F, Cl, Br
• R₄: H oder organischer verzweigter und unverzweigter Alkyl- oder Aryl-Substituent (oder Kombinationen von beiden) mit bis zu 30 Atomen ausgewählt aus C, H, Si, O, N, P, S, F, Cl, Br oder weitere Nitrogruppe, Alkoxygruppe oder Nitrilgruppe
• X: O, S, NH, NR (R ist ein organischer verzweigter oder unverzweigter Alkyl-oder Aryl-Substituenten (oder Kombinationen von beiden) mit bis zu 30 Atomen ausgewählt aus C, H, Si, O, N, P, S, F, Cl, Br.

6. Ophthalmologische Zusammensetzung nach Anspruch 5, **dadurch gekennzeichnet, dass** R₂ und R₃ = C₂H₄, X = O, R₁ = Acryl- oder Methacrylrest, R₄ = H mit der Struktur: 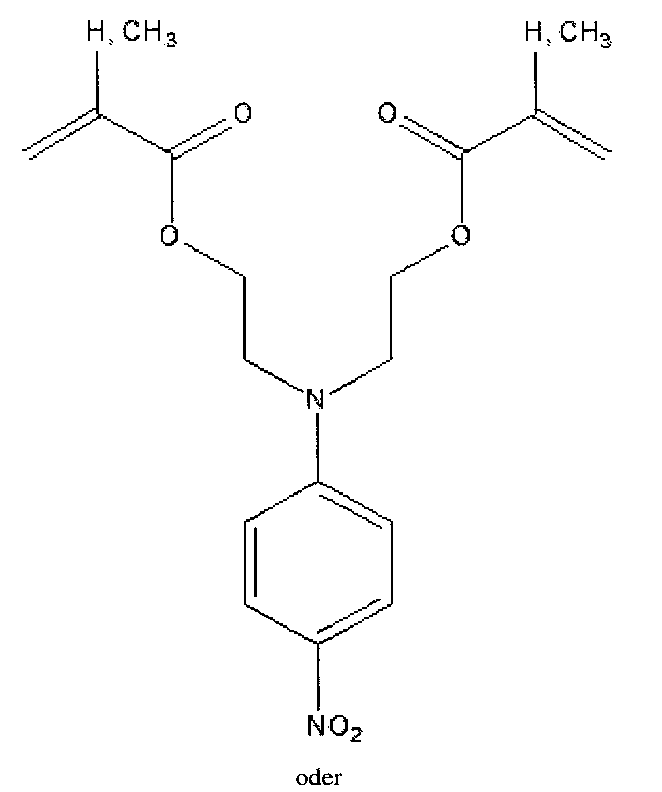 R₂ und R₃ = -CH₂-CH(CH₃)-, X = O, R₁ = Acryl- oder Methacrylrest, R₄ = H mit der Struktur: 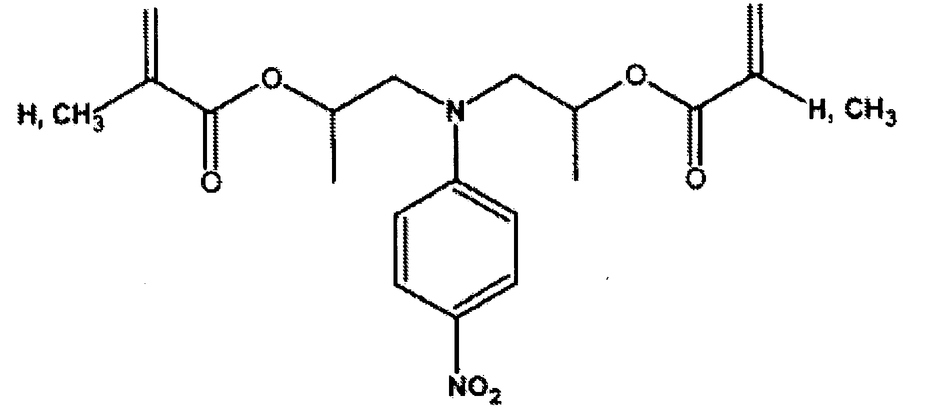 oder
R₂ und R₃ = C₂H₄, X = NR, R₁ = Acryl- oder Methacrylrest, R₄ = H mit der Struktur: 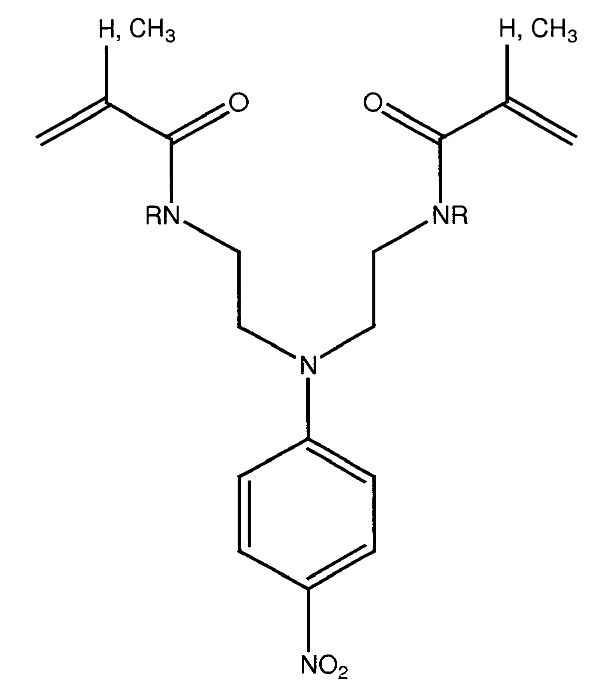 oder
R₂ und R₃ = C₂H₄, X = O, R₁ = Acryl- oder Methacrylrest, R₄ = CH₃ mit der Struktur: 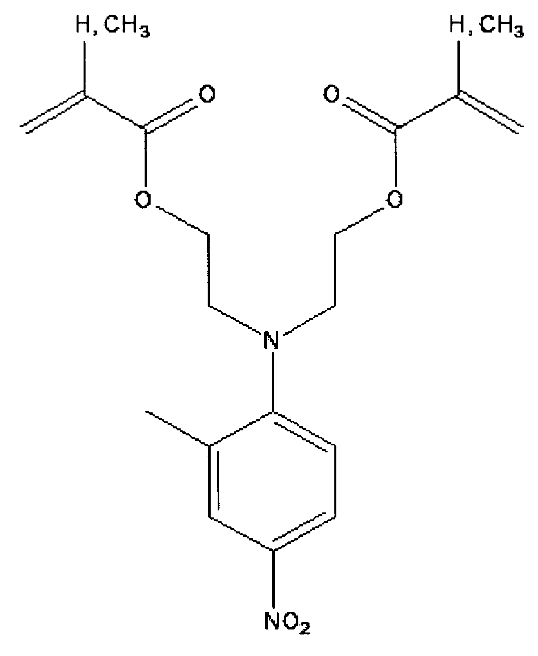 ist.

7. Ophthalmologische Zusammensetzung nach einem der Ansprüche 1 bis 6, **dadurch gekennzeichnet, dass** der Violett-Absorber ein Stereoisomer oder ein racemisches Gemisch von Farbstoffen folgender Struktur ist: 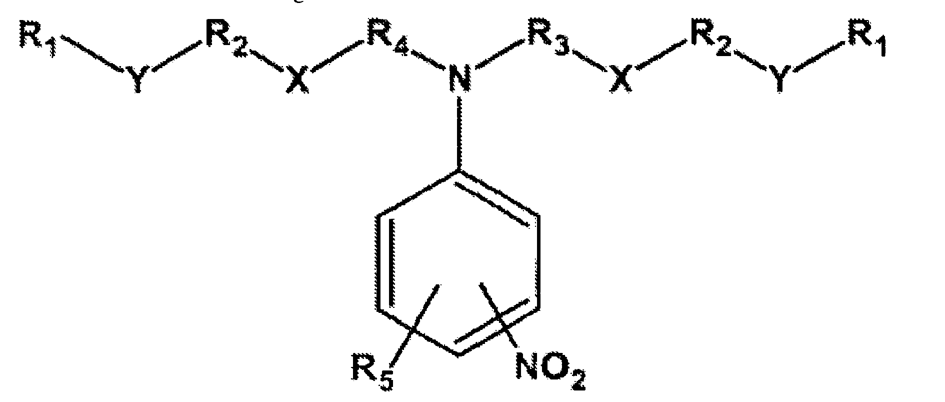 wobei
• R₁ ein Acryl- bzw. Methacryl-Rest ist
• R₂: organische verzweigte und unverzweigte Alkyl- oder Arylspacergruppen (oder Kombinationen von beiden) mit bis zu 30 Atomen ausgewählt aus C, H, Si, O, N, P, S, F, Cl, Br
• R₃: organische verzweigte und unverzweigte Alkyl- oder Arylspacergruppen (oder Kombinationen von beiden) mit bis zu 30 Atomen ausgewählt aus C, H, Si, O, N, P, S, F, Cl, Br
• R₄: organischer verzweigter und unverzweigter Alkyl- oder Aryl-Substituent (oder Kombinationen von beiden) mit bis zu 30 Atomen ausgewählt aus C, H, Si, O, N, P, S, F, Cl, Br
• R₅: H oder organischer verzweigter und unverzweigter Alkyl- oder Aryl-Substituent (oder Kombinationen von beiden) mit bis zu 30 Atomen ausgewählt aus C, H, Si, O, N, P, S, F, Cl, Br oder weitere Nitrogruppe, Alkoxygruppe oder Nitrilgruppe
• X, Y: O, S, NH, NR (R ist ein organischer verzweigter oder unverzweigter Alkyl- oder Aryl-Substituenten (oder Kombinationen von beiden) mit bis zu 30 Atomen ausgewählt aus C, H, Si, O, N, P, S, F, Cl, Br.

8. Ophthalmologische Zusammensetzung nach Anspruch 7, **dadurch gekennzeichnet, dass** R₃ und R₄ = C₂H₄, X = O, R₂ = -CH₂-CH(OH)CH₂-, Y = O, R₁ = Acryl- oder Methaciylrest, R₅ = H mit der Struktur: 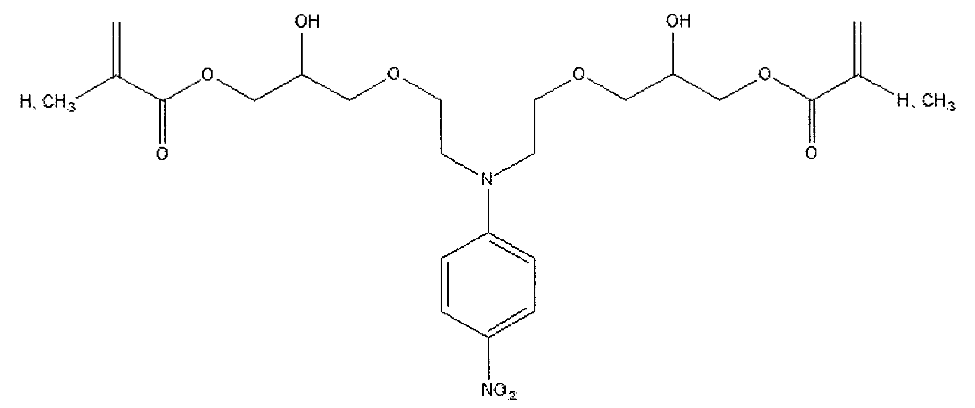 ist.

9. Ophthalmologische Zusammensetzung nach einem der Ansprüche 1 bis 8, **gekennzeichnet durch** eine biokompatible Trägersubstanz, in welcher der UV-Absorber und der Violett-Absorber vorgesehen sind, wobei die die Trägersubstanz aus mindestens einem Acrylat besteht.

10. Ophthalmologische Zusammensetzung nach Anspruch 9, **dadurch gekennzeichnet, dass** der UV-Absorber und der Violett-Absorber kovalent im Acrylat-Material gebunden sind und/oder dass wenigstens ein Acrylat ein hydrophiles Material mit einem Wassergehalt von 1% bis 30% ist und/oder dass wenigstens ein Acrylat HEMA und/oder MMA sind bzw. ist.

11. Ophthalmologische Zusammensetzung nach einem der Ansprüche 1 bis 10, als Trägermaterial enthaltend:
| | |
|---|---|
| EOEMA (Ethoxyethylmethacrylat) | 85 - 97 Gew.-% |
| MMA (Methylmethacrylat) | 0-15 Gew.-% |
| EEEA (Ethoxyethoxyethylacrylat) | 0 - 5 Gew.-% |
| EGDMA (Ethylenglykoldimethacrylat) | 0 - 0,7 Gew.-% |
| UV-Absorber | 0,1-1,0 Gew.-% |
| Violett-Absorber | 0,03 - 0,16 Gew.-% |

12. Ophthalmologische Zusammensetzung nach einem der Ansprüche 1 bis 10, als Trägermaterial enthaltend
| | |
|---|---|
| HEMA (Hydroxyethylmethacrylat) | 50 - 85 Gew.-% |
| EOEMA (Ethoxyethylmethacrylat) | 30 - 40 Gew.-% |
| THFMA (Tetrahydrofufurylmethacrylat) | 5-20 Gew.-% |
| EGDMA (Ethylenglykoldimethacrylat) | 0 - 0,7 Gew.-% |
| UV-Absorber | 0,1-1,0 Gew.-% |
| Violett-Absorber | 0,03 -0,16 Gew.-% |

13. Verwendung einer ophthalmologischen Zusammensetzung nach einem der Ansprüche 1 bis 12 zur Herstellung einer ophthalmischen Linse, eines ophthalmologischen Implantats oder einer Intraokularlinse.

14. Augenimplantat, insbesondere Intraokularlinse, dessen Implantatmaterial eine ophthalmologische Zusammensetzung nach einem der Ansprüche 1 bis 12 aufweist.

## Claims

1. Ophthalmologic composition, which is constructed based on acrylate and/or methacrylate and which has an UV absorber, in which one or more acrylic acid or methacrylic acid units are bound to a substituted or unsubstituted coumarin base structure via an alkyl spacer, as well as contains a violet absorber (yellow dye) based on an N-alkoxyacrylated or N-alkoxymethacrylated or based on an N,N-dialkoxyacrylated or N,N-dialkoxymethacrylated substituted or unsubstituted nitroanline.

2. Ophthalmologic composition according to claim 1, in which the UV absorber is a stereoisomer or a racemic mixture of the following structure: 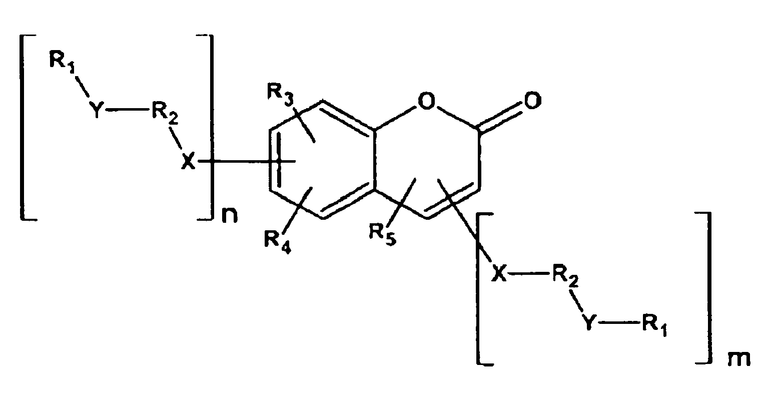 wherein
• R₁ is an acryl or methacryl radical
• R₂: organic branched and unbranched alkyl or aryl substituents (or combinations of both) with up to 30 atoms selected from C, H, Si, O, N, P, S, F, Cl, Br
• R₃, R₄ and R₅: H or organic branched and unbranched alkyl or aryl substituents (or combinations of both) with up to 30 atoms selected from C, H, Si, O, N, P, S, F, Cl, Br
• X and Y: O, S, NH, NR (R is an organic branched or unbranched alkyl or aryl substituent (or combinations of both) with up to 30 atoms selected from C, H, Si, O, N, P, S, F, Cl, Br
• n = 0 to 2 as well as m = 0 or 1, wherein n+m is always greater than or equal to 1.

3. Ophthalmologic composition according to claim 2, **characterized in that** n = 1, m=0, X = O, R₂ = C₃H₆, Y = O, R₁ = acryl or methacryl radical, R₃ = H, R₄ = H, R₅ = H with the structure: 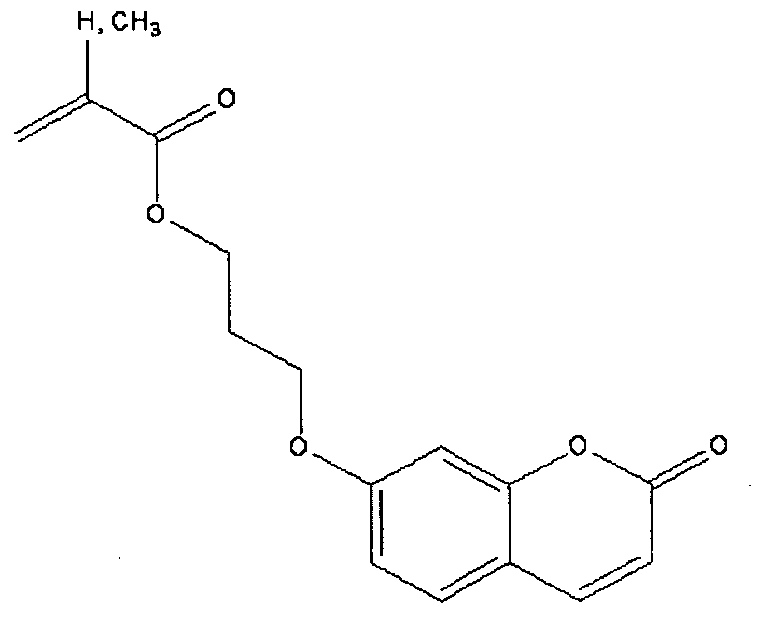 or
n = 2, m = 0, X = O, R₂ = C₃H₆, Y = O, R₁ = acryl or methacryl radical, R₃ = H, R₄ = H, R₅ = H with the structure: 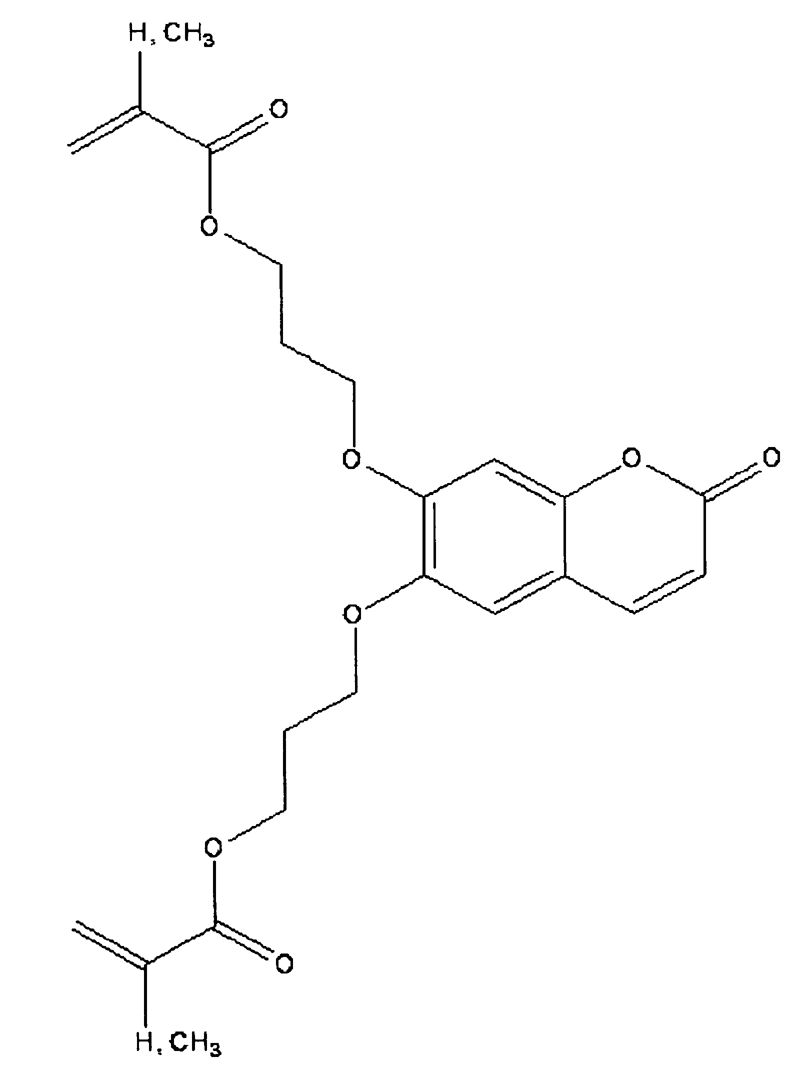 or
n = 1, m = 0, X = O, R₂ = -CH₂-CH(OR₁)CH₂-, Y = O, R₁ = acryl or methacryl radical, R₃ = H, R₄ = H, R₅ = H with the structure:

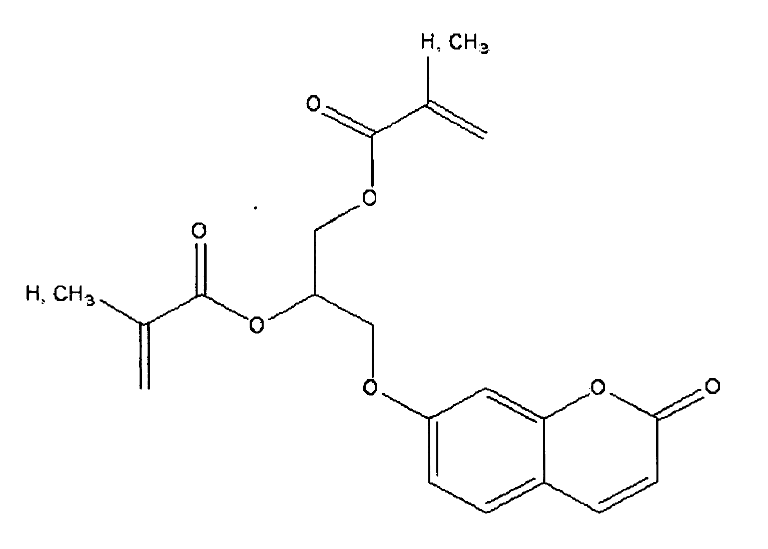

4. Ophthalmologic composition according to claim 2, **characterized in that** n = 1, m=0, X = O, R₂ = -CH₂-CH(OH)CH₂-, Y = O, R₁ = acryl or methacryl radical, R₃ = H, R₄ = H, R₅ = H with the structure: 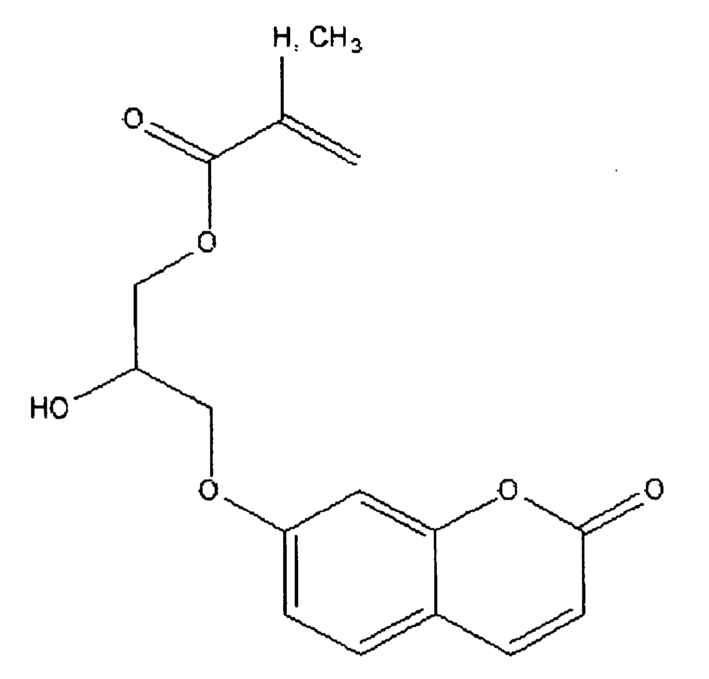 or
n = 1, m = 0, X = O, R₂ = C₃H₆, Y = O, R₁ = acryl or methacryl radical, R₃ = H, R₄ = H, R₅ = C₃H₇ with the structure: 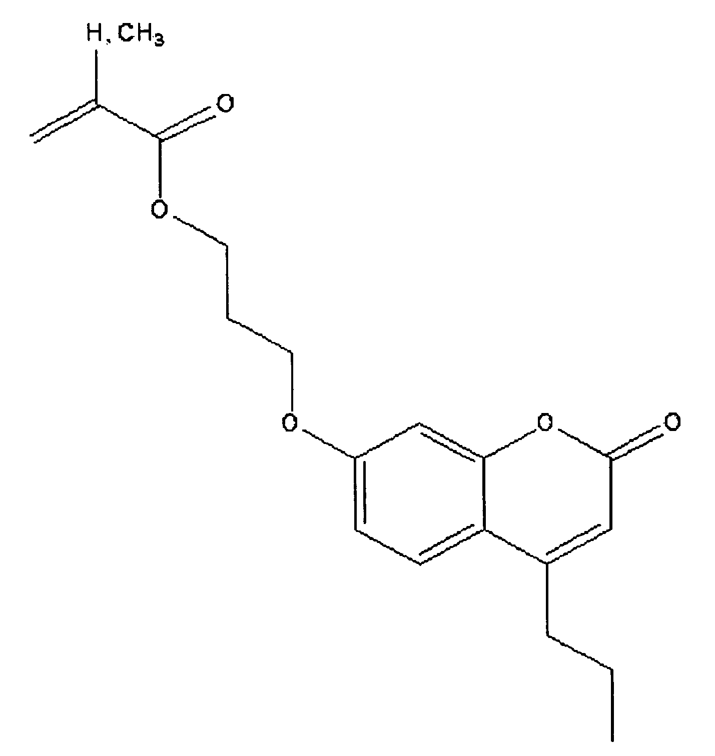 or
n = 2, m = 1, X = O, R₂ = C₃H₆, Y = O, R₁ = acryl or methacryl radical, R₃ = H, R₄ = H, R₅ = H with the structure:

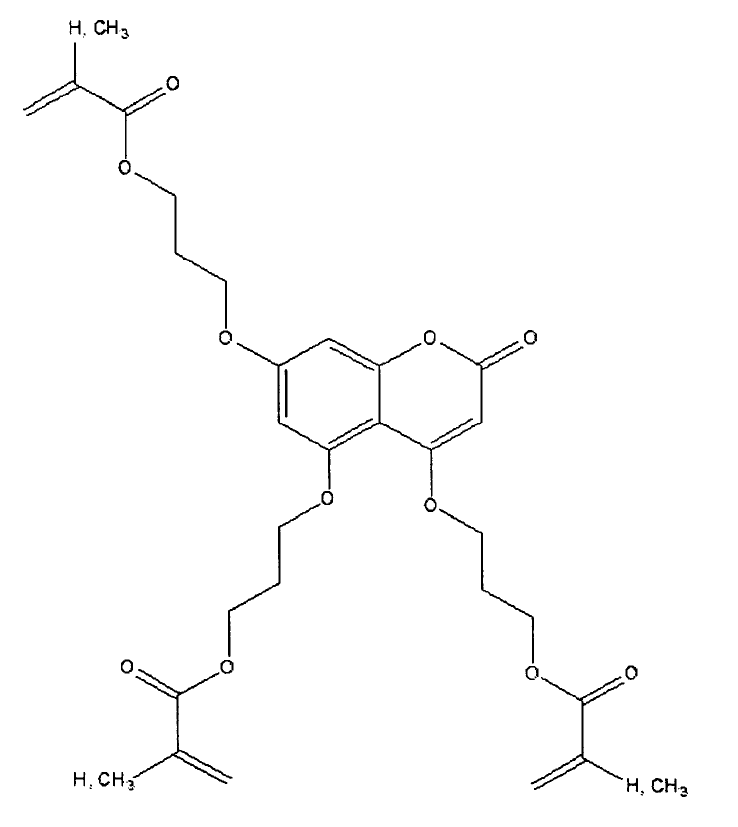

5. Ophthalmologic composition according to any one of claims 1 to 4, **characterized in that** the violet absorber is a stereoisomer or a racemic mixture of dyes of the following structure: 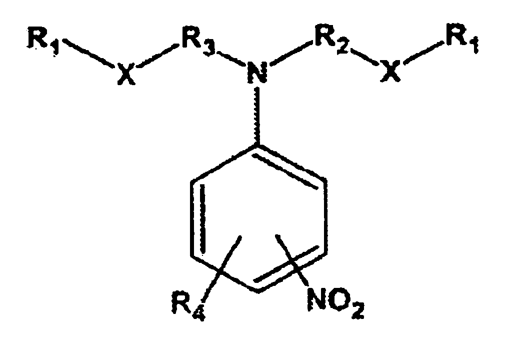 wherein
• R₁ is an acryl or methacryl radical
• R₂: organic branched and unbranched alkyl or aryl spacer group (or combinations of both) with up to 30 atoms selected from C, H, Si, O, N, P, S, F, Cl, Br
• R₃: organic branched and unbranched alkyl or aryl substituent (or combinations of both) with up to 30 atoms selected from C, H, Si, O, N, P, S, F, Cl, Br
• R₄: H or organic branched and unbranched alkyl or aryl substituent (or combinations of both) with up to 30 atoms selected from C, H, Si, O, N, P, S, F, Cl, Br or further nitro group, alkoxy group or nitrile group
• X: O, S, NH, NR (R is an organic branched or unbranched alkyl or aryl substituent (or combinations of both) with up to 30 atoms selected from C, H, Si, O, N, P, S, F, Cl, Br.

6. Ophthalmologic composition according to claim 5, **characterized in that** R₂ and R₃ = C₂H₄, X = O, R₁ = acryl or methacryl radical, R₄ = H with the structure: 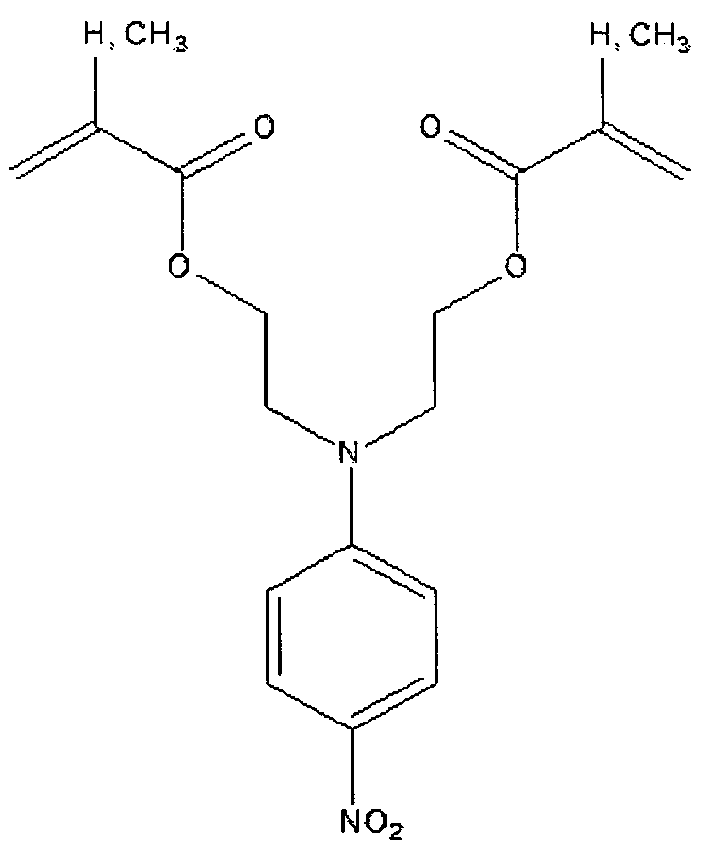 or
R₂ and R₃ = -CH₂-CH(CH₃)-, X = O, R₁ = acryl or methacryl radical, R₄ = H with the structure: 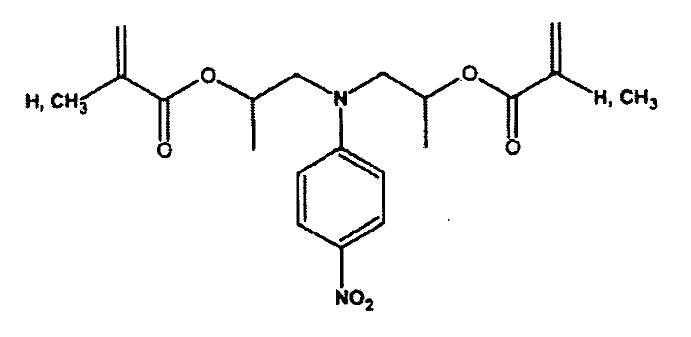 or
R₂ and R₃ = C₂H₄, X = NR, R₁ = acryl or methacryl radical, R₄ = H with the structure: 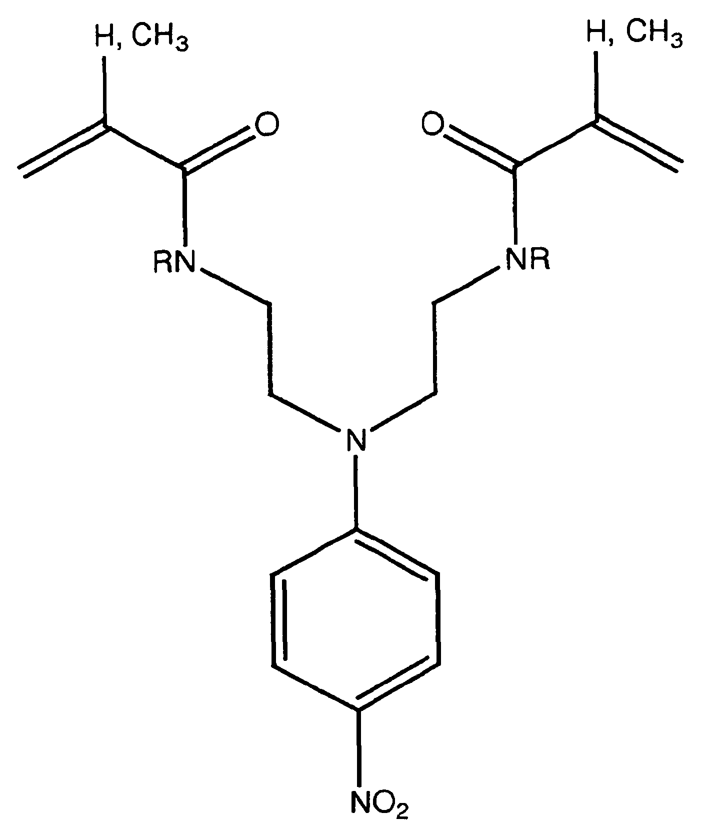 or
R₂ and R₃ = C₂H₄, X = O, R₁ = acryl or methacryl radical, R₄ = CH₃ with the structure:

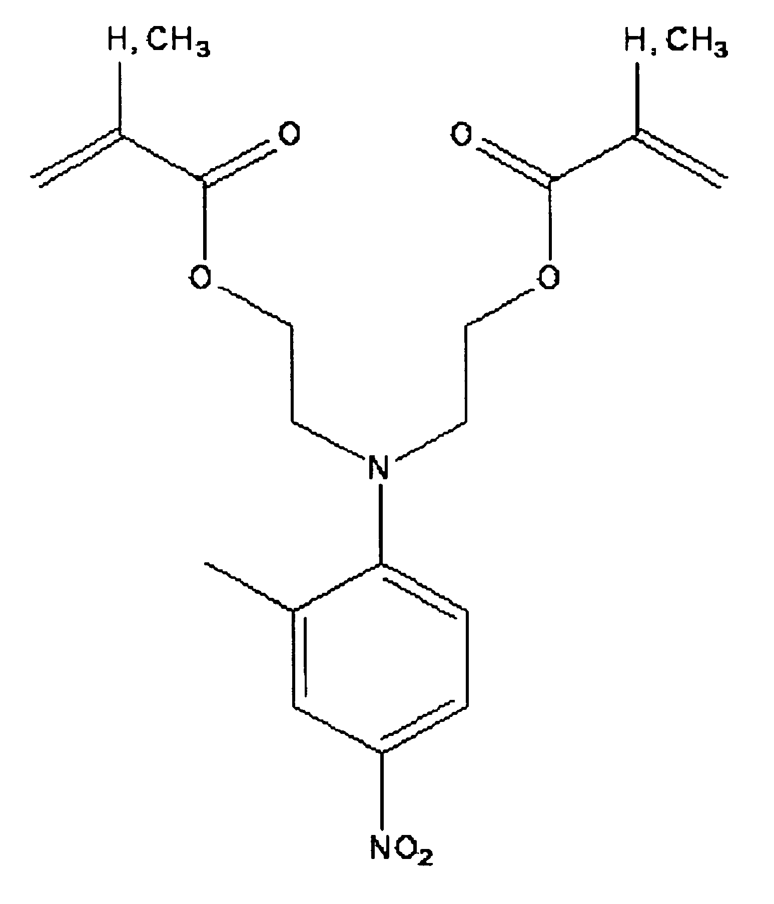

7. Ophthalmologic composition according to any one of claims 1 to 6, **characterized in that** the violet absorber is a stereoisomer or a racemic mixture of dyes of the following structure: 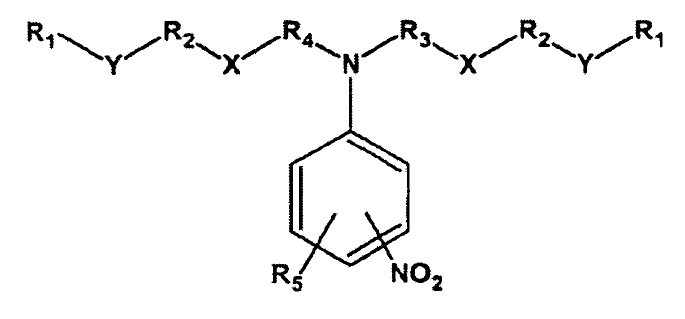 wherein
• R₁ is an acryl or methacryl radical
• R₂: organic branched and unbranched alkyl or aryl spacer groups (or combinations of both) with up to 30 atoms selected from C, H, Si, O, N, P, S, F, Cl, Br
• R₃: organic branched and unbranched alkyl or aryl spacer groups (or combinations of both) with up to 30 atoms selected from C, H, Si, O, N, P, S, F, Cl, Br
• R₄: organic branched and unbranched alkyl or aryl substituent (or combinations of both) with up to 30 atoms selected from C, H, Si, O, N, P, S, F, Cl, Br
• R₅: H or organic branched and unbranched alkyl or aryl substituent (or combinations of both) with up to 30 atoms selected from C, H, Si, O, N, P, S, F, Cl, Br or further nitro group, alkoxy group or nitrile group
• X, Y: O, S, NH, NR (R is an organic branched or unbranched alkyl or aryl substituent (or combinations of both) with up to 30 atoms selected from C, H, Si, O, N, P, S, F, Cl, Br.

8. Ophthalmologic composition according to claim 7, **characterized in that** R₃ and R₄ = C₂H₄, X = O, R₂ = -CH₂-CH(OH)CH₂-, Y = O, R₁ = acryl or methacryl radical, R₅ = H with the structure:

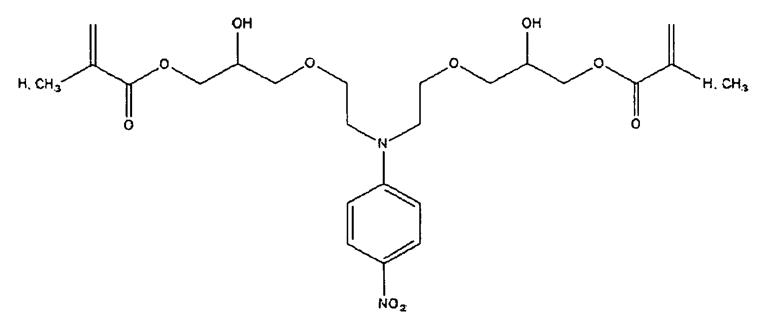

9. Ophthalmologic composition according to any one of claims 1 to 8, **characterized by** a biocompatible carrier substance, in which the UV absorber and the violet absorber are provided, wherein the carrier substance is composed of at least one acrylate.

10. Ophthalmologic composition according to claim 9, **characterized in that** the UV absorber and the violet absorber are covalently bound in the acrylate material and/or that at least one acrylate is a hydrophilic material with a water content of 1 % to 30% and/or that at least one acrylate is or are HEMA and/or MMA.

11. Ophthalmologic composition according to any one of claims 1 to 10, containing as a carrier material:
| | |
|---|---|
| EOEMA (ethoxyethyl methacrylate) | 85 - 97 % by wt. |
| MMA (methyl methacrylate) | 0-15 % by wt. |
| EEEA (ethoxyethoxy ethylacrylate) | 0 - 5 % by wt. |
| EGDMA (ethylene glycol dimethacrylate) | 0 - 0.7 % by wt. |
| UV absorber | 0.1-1.0 % by wt. |
| Violet absorber | 0.03 - 0.16 % by wt. |

12. Ophthalmologic composition according to any one of claims 1 to 10, containing as a carrier material:
| | |
|---|---|
| HEMA (hydroxyethyl methacrylate) | 50 - 85 % by wt. |
| EOEMA (ethoxyethyl methacrylate) | 30 - 40 % by wt. |
| THFMA (tetrahydrofufuryl methacrylate) | 5-20 % by wt. |
| EGDMA (ethylene glycol dimethacrylate) | 0 - 0.7 % by wt. |
| UV absorber | 0.1-1.0 % by wt. |
| Violet absorber | 0.03 -0.16 % by wt. |

13. Use of an ophthalmologic composition according to any one of claims 1 to 12 for manufacturing an ophthalmic lens, an ophthalmologic implant or an intraocular lens.

14. Eye implant, in particular intraocular lens, the implant material of which has an ophthalmologic composition according to any one of claims 1 to 12.

## Revendications

1. Composition ophtalmologique constituée à base d'acrylates et/ou de méthacrylates et présentant un absorbeur d'UV dont une ou plusieurs unités d'acide acrylique respectivement d'acide méthacrylique sont liées au moyen d'un espaceur alkylique à une structure de base de coumarine substituée ou non substituée, ainsi qu'un absorbeur de violets (colorant jaune) sur la base d'une nitroaniline N-alkoxy-acrylée respectivement N-alkoxy-méthacrylée ou sur la base d'une nitroaniline N,N-dialkoxy-acrylé respectivement N,N-dialkoxy-méthacrylé substituée ou non substituée.

2. Composition ophtalmologique selon la revendication 1, **caractérisée en ce que** l'absorbeur d'UV est un stéréoisomère ou un mélange racémique présentant la structure suivante 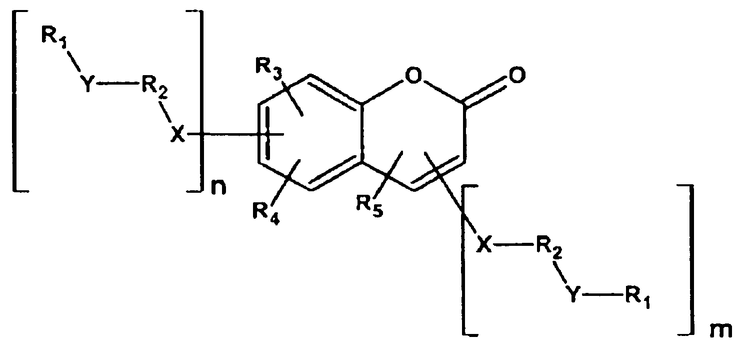 dans laquelle :
• R1 est un groupement acrylique respectivement méthacrylique
• R2 : des substituants alkyliques ou aryliques organiques ramifiés et non ramifiés (ou des combinaisons entre les deux) comprenant jusqu'à 30 atomes choisis parmi C, H, Si, O, N, P, S, F, Cl, Br
• R3, R4 et R5 : H ou des substituants alkyliques ou aryliques organiques ramifiés et non ramifiés (ou des combinaisons entre les deux) comprenant jusqu'à 30 atomes choisis parmi C, H, Si, O, N, P, S, F, Cl, Br
• X et Y : O, S, NH, NR (R étant un substituant alkylique ou arylique organique ramifié ou non ramifié (ou des combinaisons entre les deux) comprenant jusqu'à 30 atomes choisis parmi C, H, Si, O, N, P, S, F, Cl, Br
• n = 0 à 2 ainsi que m = 0 ou 1, n + m toujours étant égal ou supérieur à 1.

3. Composition ophtalmologique selon la revendication 2, **caractérisée en ce que** n = 1, m=0, X = O, R₂ = C₃H₆, Y = O, R₁ = groupement acrylique respectivement méthacrylique, R₃ = H, R₄ = H, R₅ = H, la structure étant la suivante : 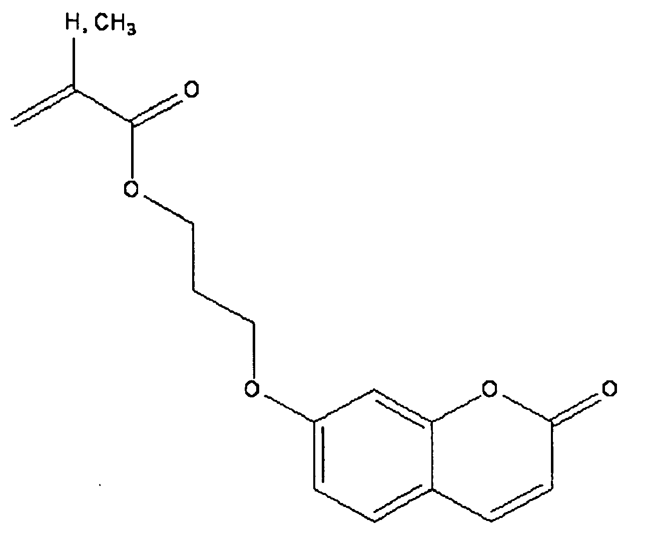 ou :
n = 2, m=0, X = O, R₂ = C₃H₆, Y = O, R₁ = groupement acrylique respectivement méthacrylique, R₃ = H, R₄ = H, R₅ = H, la structure étant la suivante : 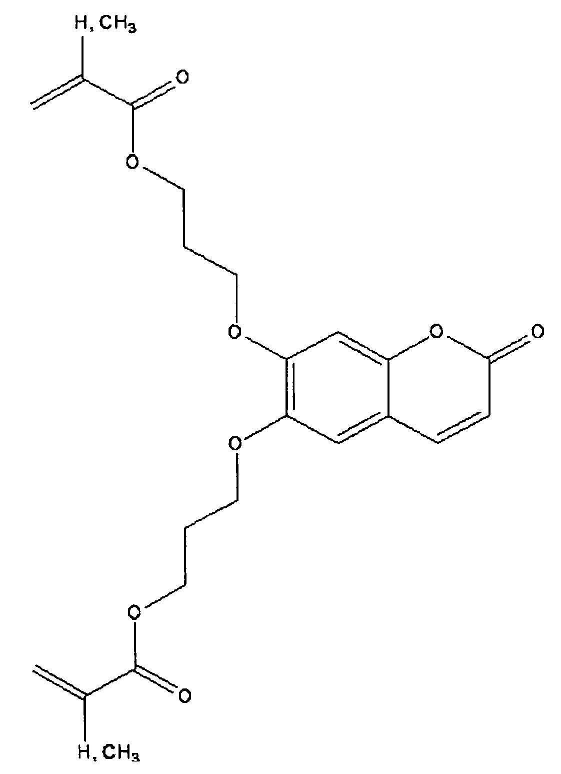
ou :
n = 1, m=0, X = O, R₂ = -CH₂-CH(OR₁)CH₂-, Y = O, R₁ = groupement acrylique respectivement méthacrylique, R₃ = H, R₄ = H, R₅ = H, la structure étant la suivante :

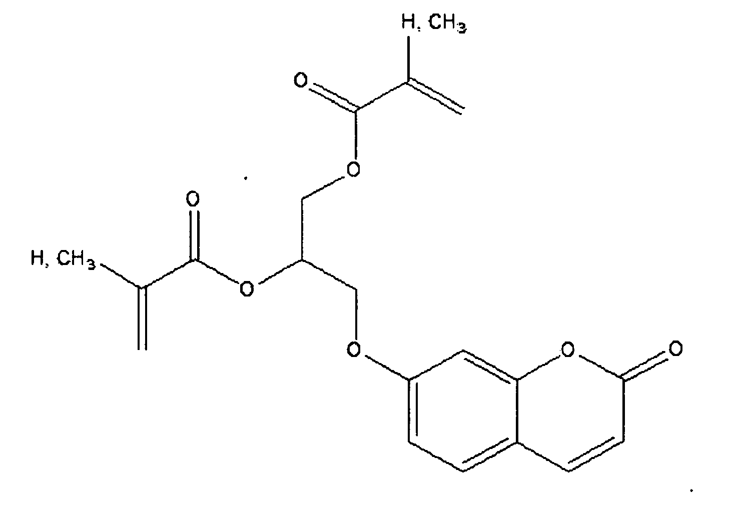

4. Composition ophtalmologique selon la revendication 2, **caractérisée en ce que** n = 1, m = 0, X = O, R₂ = -CH₂-CH(OH)CH₂-, Y = O, R₁ = groupement acrylique respectivement méthacrylique, R₃ = H, R₄ = H, R₅ = H, la structure étant la suivante : 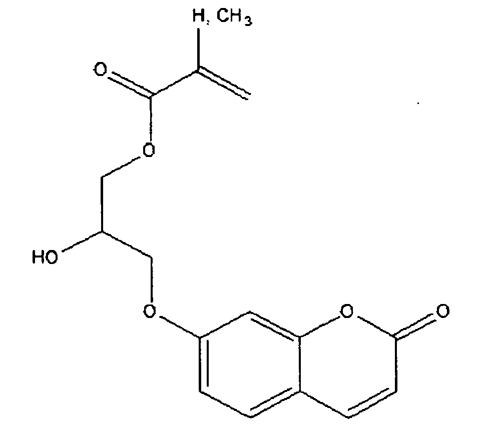 ou :
n = 1, m = 0, X = O, R₂ = C₃H₆, Y = O, R₁ = groupement acrylique respectivement méthacrylique, R₃ = H, R₄ = H, R₅ = C₃H₇, la structure étant la suivante : 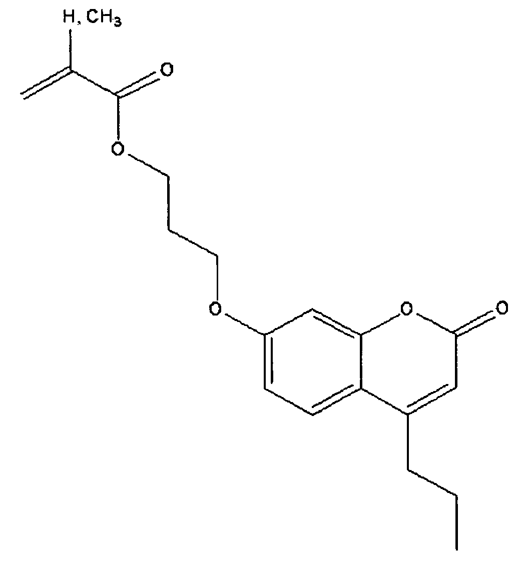
ou :
n = 2, m = 1, X = O, R₂ = C₃H₆, Y = O, R₁ = groupement acrylique respectivement méthacrylique, R₃ = H, R₄ = H, R₅ = H, la structure étant la suivante :

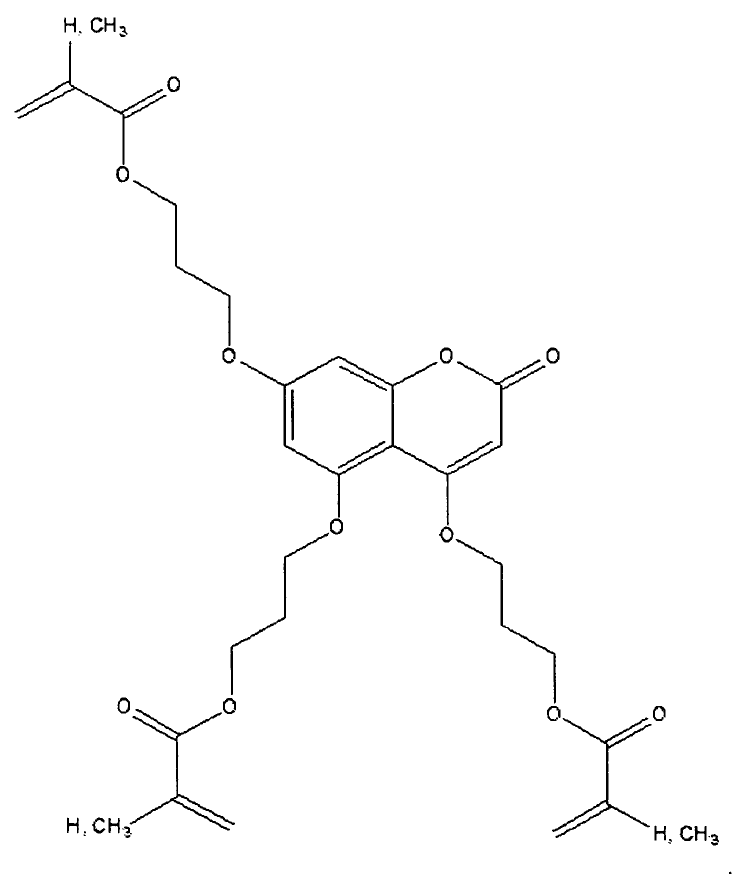

5. Composition ophtalmologique selon l'une des revendications 1 à 4, **caractérisée en ce que** l'absorbeur de violets est un stéréoisomère ou un mélange racémique de colorants dont la structure est la suivante 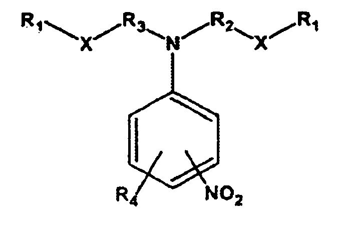 dans laquelle :
• R1 est un groupement acrylique respectivement méthacrylique
• R2 : un groupement espaceur alkylique ou arylique organique ramifié et non ramifié (ou des combinaisons entre les deux) comprenant jusqu'à 30 atomes choisis parmi C, H, Si, O, N, P, S, F, Cl, Br
• R3 : un substituant alkylique ou arylique organique ramifié et non ramifié (ou des combinaisons entre les deux) comprenant jusqu'à 30 atomes choisis parmi C, H, Si, O, N, P, S, F, Cl, Br
• R4 : H ou un substituant alkylique ou arylique organique ramifié et non ramifié (ou des combinaisons entre les deux) comprenant jusqu'à 30 atomes choisis parmi C, H, Si, O, N, P, S, F, Cl, Br, ou un autre groupement nitro, un groupement alkoxy ou un groupement nitrile
• X : O, S, NH, NR (R étant un substituant alkylique ou arylique organique ramifié ou non ramifié (ou des combinaisons entre les deux) comprenant jusqu'à 30 atomes choisis parmi C, H, Si, O, N, P, S, F, Cl, Br.

6. Composition ophtalmologique selon la revendication 5, **caractérisée en ce que** R₂ et R₃ = C₂H₄, X = O, R₁ = groupement acrylique respectivement méthacrylique, R₄ = H, la structure étant la suivante : 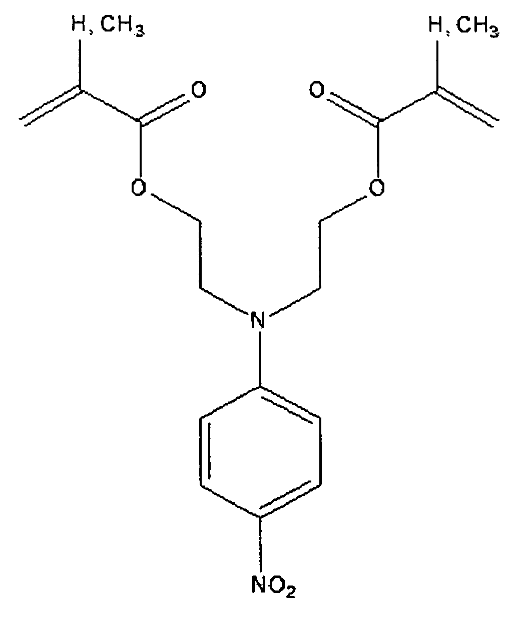 ou :
R₂ et R₃ = -CH₂-CH(CH₃)-, X = O, R₁ = groupement acrylique ou méthacrylique, R₄ = H, la structure étant la suivante : 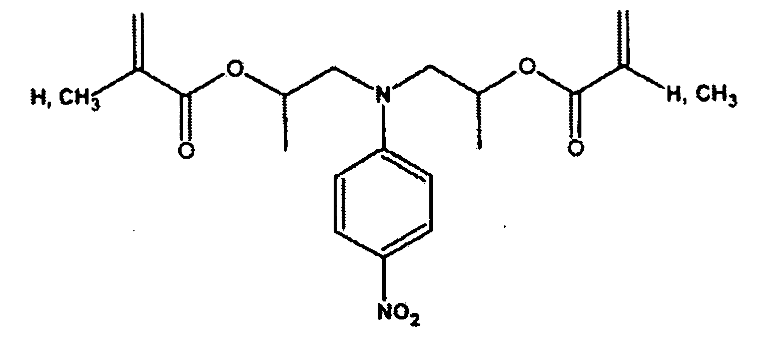
ou :
R₂ et R₃ = C₂H₄, X = NR, R₁ = groupement acrylique respectivement méthacrylique, R₄ = H, la structure étant la suivante : 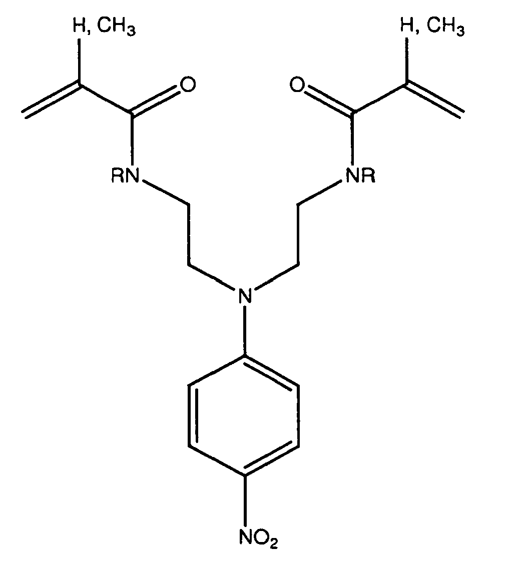
ou :
R₂ et R₃ = C₂H₄, X = O, R₁ = groupement acrylique respectivement méthacrylique, R₄ = CH₃, la structure étant la suivante :

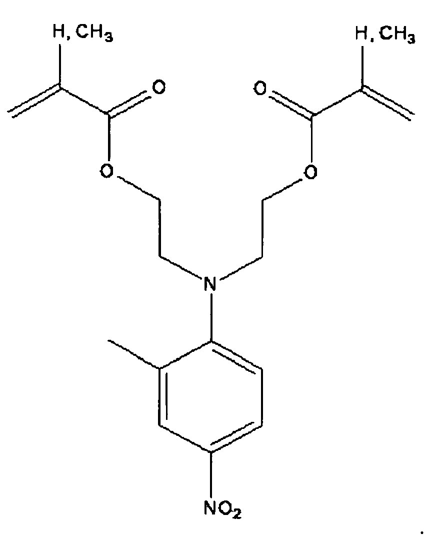

7. Composition ophtalmologique selon l'une des revendications 1 à 6, **caractérisée en ce que** l'absorbeur de violets est un stéréoisomère ou un mélange racémique de colorants dont la structure est la suivante : 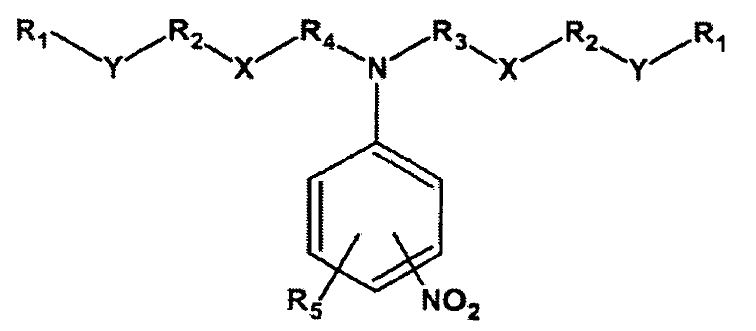 dans laquelle :
• R1 est un groupement acrylique respectivement méthacrylique
• R2 : des groupements espaceurs alkyliques ou aryliques organiques ramifiés et non ramifiés (ou des combinaisons entre les deux) comprenant jusqu'à 30 atomes choisis parmi C, H, Si, O, N, P, S, F, Cl, Br
• R3 : des groupements espaceurs alkyliques ou aryliques organiques ramifiés et non ramifiés (ou des combinaisons entre les deux) comprenant jusqu'à 30 atomes choisis parmi C, H, Si, O, N, P, S, F, CI, Br
• R4 : un substituant alkylique ou arylique organique ramifié et non ramifié (ou des combinaisons entre les deux) comprenant jusqu'à 30 atomes choisis parmi C, H, Si, O, N, P, S, F, CI, Br
• R5 : H ou un substituant alkylique ou arylique organique ramifié et non ramifié (ou des combinaisons entre les deux) comprenant jusqu'à 30 atomes choisis parmi C, H, Si, O, N, P, S, F, Cl, Br, ou un autre groupement nitro, un groupement alkoxy ou un groupement nitrile
• X, Y : O, S, NH, NR (R étant un substituant alkylique ou arylique organique ramifié ou non ramifié (ou des combinaisons entre les deux) comprenant jusqu'à 30 atomes choisis parmi C, H, Si, O, N, P, S, F, Cl, Br.

8. Composition ophtalmologique selon la revendication 7, **caractérisée en ce que** R₃ et R₄ = C₂H₄, X = O, R₂ = -CH₂-CH(OH)CH₂-, Y = O, R₁ = groupement acrylique ou méthacrylique, R₅ = H, la structure étant la suivante :

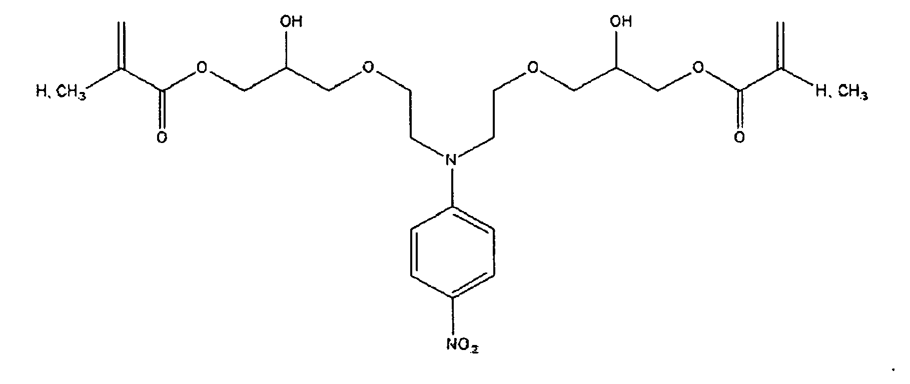

9. Composition ophtalmologique selon l'une des revendications 1 à 8, **caractérisée par** une substance porteuse dans laquelle sont prévus l'absorbeur d'UV et l'absorbeur de violets, ladite substance porteuse comprenant au moins un acrylate.

10. Composition ophtalmologique selon la revendication 9, **caractérisée en ce que** l'absorbeur d'UV et l'absorbeur de violets sont liés de manière covalente dans l'acrylate et/ou qu'au moins l'un des acrylates est un matériau hydrophile dont la teneur en eau se situe entre 1% jusqu'à 30% et/ou **en ce qu'**au moins l'un des acrylates est HEMA et/ou MMA.

11. Composition ophtalmologique selon l'une des revendications 1 à 10, comprenant comme substances porteuses :
| | |
|---|---|
| EOEMA (méthacrylate d'éthoxyéthyle) : | 85 à 97 % en poids |
| MMA (méthacrylate de méthyle) : | 0 à 15 % en poids |
| EEEA (éthoxy-éthoxy-acrylate d'éthyle) : | 0 à 5 % en poids |
| EGDMA (diméthacrylate d'éthylène glycol) : | 0 à 0,7 % en poids |
| Absorbeur d'UV : | 0,1 à 1,0 % en poids |
| Absorbeur de violets : | 0,03 à 0,16 % en poids. |

12. Composition ophtalmologique selon l'une des revendications 1 à 10, comprenant comme substances porteuses :
| | |
|---|---|
| HEMA (méthacrylate d'hydroxyéthyle) : | 50 à 85 % en poids |
| EOEMA (méthacrylate d'éthoxyéthyle) : | 30 à 40 % en poids |
| THFMA (méthacrylate de tétrahydrofurfuryle) : | 5 à 20 % en poids |
| EGDMA (diméthacrylate d'éthylène glycol) : | 0 à 0,7 % en poids |
| Absorbeur d'UV : | 0,1 à 1,0 % en poids |
| Absorbeur de violets : | 0,03 à 0,16 % en poids. |

13. Utilisation d'une composition ophtalmologique selon l'une des revendications 1 à 12 pour la fabrication d'une lentille ophtalmique, d'un implant ophtalmologique et d'une lentille intra-oculaire.

14. Implant oculaire, en particulier lentille intra-oculaire, le matériau dudit implant présentant une composition ophtalmologique selon l'une des revendications 1 à 12.
